# EUROPEAN PATENT SPECIFICATION

(11) **EP 3 134 733 B1**
(45) Date of publication and mention of the grant of the patent: **14.10.2020**
(21) Application number: 15782472.3
(22) Date of filing: 23.04.2015
(51) Int. Cl.: C12Q 1/6883, G01N 33/68

(54) **ASSAY AND METHOD FOR TREATING SUBJECTS WITH IMMUNE-MEDIATED DISEASES**
TEST UND VERFAHREN ZUR BEHANDLUNG VON PATIENTEN MIT IMMUNVERMITTELTEN ERKRANKUNGEN
TEST ET PROCÉDÉ POUR TRAITER DES PATIENTS AYANT DES MALADIES À MÉDIATION IMMUNITAIRE

(30) Priority: 25.04.2014 US 201461984652 P
(43) Date of publication of application: 01.03.2017
(73) Proprietor: The Brigham and Women's Hospital, Inc., Boston, MA 02115 (US)
(72) Inventor: BLUMBERG, Richard S., Waltham Massachusetts 02451 (US); BAKER, Kristi, Edmonton, Alberta T6G 1H7 (CA); RATH, Timo, 91056 Erlangen (DE)
(74) Representative: Barker Brettell LLP
(86) International application number: PCT/US2015/027295
(87) International publication number: WO 2015/164605

(56) References cited:
- KR-A- 20120 099 862
- US-A1- 2005 075 306
- US-A1- 2005 260 680
- US-A1- 2006 165 653
- US-A1- 2010 254 971
- US-A1- 2010 266 530
- US-A1- 2010 291 549
- US-A1- 2010 291 549
- US-A1- 2012 094 291
- US-A1- 2013 078 262
- US-A1- 2014 105 913
- XINRUI LI ET AL: "Targeting the Fc receptor in autoimmune disease", EXPERT OPINION ON THERAPEUTIC TARGETS, vol. 18, no. 3, 13 February 2014 (2014-02-13), pages 335-350, XP055292652, UK ISSN: 1472-8222, DOI: 10.1517/14728222.2014.877891
- Wl Van Der Pol ET AL: "IgG receptor IIa alleles determine susceptibility and severity of Guillain-Barré syndrome", Neurology, 25 April 2000 (2000-04-25), pages 1661-1665, XP055404154, Retrieved from the Internet: URL:http://www.neurology.org/content/54/8/ 1661.full.pdf#page=1&view=FitH [retrieved on 2017-09-06]

## Description

### CROSS-REFERENCE TO RELATED APPLICATION

This application claims benefit under 35 U.S.C. § 119(e) of U.S. Provisional Application No. 61/984,652 filed April 25, 2014.

### GOVERNMENT RIGHTS

The invention was made with government support under Grant No. DK53056 awarded by the National Institutes of Health. The government has certain rights to the invention.

### FIELD OF INVENTION

Described herein are assays, methods and systems for selecting patients with immune-mediated diseases characterized by elevated IgG levels that are suitable for anti-FcRn therapy and treating the selected subjects with anti-FcRn therapy. Also described herein are methods for assessing efficacy of anti-FcRn treatment in a subject by monitoring the levels of the IgG.

### BACKGROUND OF THE INVENTION

The following description includes information that may be useful in understanding the present invention. It is not an admission that any of the information provided herein is prior art or relevant to the presently claimed invention, or that any publication specifically or implicitly referenced is prior art.

Many immune-mediated diseases are characterized by increased levels of immunoglobulin G (IgG). There are a wide variety of diseases that possess this property. These include, for example, rheumatoid arthritis, systemic lupus erythematosus, pemphigous vulgaris, and inflammatory bowel disease (IBD) among others. IBD is a prototype of such diseases, as high levels of IgG are commonly observed, and decreasing the IgG levels is therefore desirable in treating the disease. IBD involves chronic inflammation of all or part of the digestive tract. IBD primarily includes ulcerative colitis and Crohn's disease. IBD is often painful and debilitating, and sometimes leads to life-threatening complications. The existing therapies are not universally effective in treating IBD and most therapies involve adverse side effects. The poor performance of existing therapy for IBD is revealed by the cumulative 1 year relapse rate of about 50% for both ulcerative colitis and Crohn's disease [Moum B, et al. Clinical course during the 1st year after diagnosis in ulcerative colitis and Crohn's disease. Results of a large, prospective population- based study in southeastern Norway 1990-93. Scand J Gastroenterol. 1997;32:1005-1012]. With the exception of colectomy for ulcerative colitis, itself a procedure with long-term morbidity in many patients, no therapy offers a cure. [Carty, E and Rampton, DS., Evaluation of new therapies for inflammatory bowel disease. Br J Clin Pharmacol. 2003 October; 56(4): 351-361]. In view of the limitations of the existing treatments and given that not all IBD therapies are universally effective in treating IBD, there is a need in the art for assays and systems for selecting IBD subjects on whom therapies are likely to be successful and administering the therapies to the selected subjects to treat IBD. US 2010/291549 A1 describes methods and materials for determining the responsiveness of a subject to an anti-CD20 therapy based on a FcγR2a polymorphism.

### SUMMARY OF THE INVENTION

The following embodiments and aspects thereof are described and illustrated in conjunction with systems, compositions and methods which are meant to be exemplary and illustrative, not limiting in scope.

As demonstrated herein, in part via co-immunoprecipitation of FcgR and FcRn in the presence of an FcRn competent and incompetent IgG, the more signaling that occurs through either receptor FcgR and FcRn, the more biochemical interactions (and thus dependencies) there exist between these two molecules. Accordingly, as demonstrated herein, FcgR2aHis variants are more responsive to FcRn blockade.

In one aspect of the invention there is provided an assay comprising (i) assaying a biological sample obtained from a subject suspected of having an immune-mediated disease characterised by elevated IgG levels to determine the allele at position 131 of the FcγR2a protein; and (ii) selecting the subject for a therapy if the subject expresses the His131 (also known as His167 allele according to NM_001136219.1) allele of FcγR2a, wherein the therapy comprises an antibody that blocks the neonatal Fc receptor (FcRn). In some embodiments, the subject is human.

In another aspect of the invention there is provided a composition comprising an anti-FcRn therapy comprising an antibody that blocks the neonatal Fc receptor (FcRn) for use in treating, inhibiting and/or reducing the severity of an immune-mediated disease characterised by elevated IgG levels in a subject, wherein the subject has been identified as having the His131 allele of FcγR2a by the assay of the invention.

In various embodiments, the immune-mediated disease includes, but is not limited to, any one or more of monoclonal gammopathy of unknown significance (MGUS), inflammatory bowel disease, pemphigus or pemphigoid, myasthenia gravis, autoimmune hemolytic anemia, Kawasaki's disease, multiple myeloma, rheumatoid arthritis, systemic lupus erythematosus (SLE), cirrhosis of the liver, chronic hepatitis, chronic infection, multiple sclerosis (MS), macroglobulinemia, viral hepatitis, mononucleosis, kidney damage (nephrotic syndrome), celiac diseasae, allergic reactions, asthma, atopic dermatitis, cancer, idiopathic thrombocytopenic purpura (ITP), autoimmune thrombocytopenia, immune neutropenia, antiphospholipid syndrome, ANCA-associated disease, polymyositis, myasthenia gravis, diabetes, autoimmune thyroiditis, optic neuritis, Graves' opthalmopathy, Guillian-Barre Syndrome, chronic polyneuropathy, acute myeloid leukemia or a combination thereof, among others.

In some embodiments, the immune mediated disease is an inflammatory bowel disease ulcerative colitis, Crohn's disease, collagenous colitis, lymphocytic colitis, ischaemic colitis, diversion colitis, Behçet's disease, indeterminate colitis or a combination thereof or another type of immune mediated disease that is characterized by elevated levels of IgG such as rheumatoid arthritis, systemic lupus erythematosus, multiple sclerosis, pemphigus vulgaris, immune thrombocytopenic purpura or autoimmune hemolytic anemia.

In some embodiments, the subject has undergone or is undergoing treatment for an immune-mediated disease, for example inflammatory bowel disease. In some aspects, the subject has not undergone treatment for inflammatory bowel disease.

In some aspects, assaying the sample comprises sequencing the gene encoding the FcγR2a receptor from the subject's sample and comparing the sequence from the subject's sample to the FcγR2a sequence in a control sample.

The subject may be heterozygous for the His131 allele of FcγR2a or homozygous for the His131 allele of FcγR2a.

In various embodiments, the antibody is selected from the group consisting of a monoclonal antibody or a fragment thereof, a polyclonal antibody or a fragment thereof, chimeric antibody, humanized antibody and single chain antibody.

### BRIEF DESCRIPTION OF THE DRAWINGS

Exemplary embodiments are illustrated in referenced figures. It is intended that the embodiments and figures disclosed herein are to be considered illustrative rather than restrictive.
**FIG. 1** depicts FcγR2a131His binds human immune complexes more efficiently than FcγR2a131Arg.
**FIG. 2** depicts FcγR2a131His mediates increased cytokine expression upon being stimulated with immune complexes compared to FcγR2a131Arg.
**FIGS. 3A-3B** depict that FcγR2a131His exhibits increased downstream signaling upon being stimulated with immune complexes compared to FcγR2a131Arg.
**FIGS. 4A-4B** depict that FcγR2a131His induces MHC II presentation and MHC I cross-presentation of immune complexed antigen more efficiently than FcγR2a131Arg.
**FIG. 5** depicts that FcγR2a131His internalizes immune complexes more efficiently than FcγR2a131Arg and transfers them into a FcRn containing compartment. GFP tagged human FcRn expressing HEK293 cells were stably transfected with FcγR2a131Arg, FcγR2a131His, or vector control and stimulated with human IgG2 containing Immune complexes for the indicated concentrations. Blue: human IgG2 Immune complex, green: GFP tagged human FcRn, red: LAMP-1.
**FIG. 6** depicts that FcRn is a critical mediator for MHC II antigen presentation and MHC I cross presentation of immune complexed antigen.
**FIG. 7** depicts that enhanced FcgR2a initiated antigen presentation is abrogated by loss of FcRn binding.
**FIG. 8** depicts that pharmacologic blockade with the DVN24 monoclonal antibody of FcRn decreases MHC II antigen presentation.
**FIGS. 9A-9D** depict that pharmacologic blockade with the DVN24 monoclonal antibody of hFcRn in hematopoietic cells protects from the development of IgG mediated colitis.
**FIG. 10** depicts that pharmacologic blockade with the DVN24 monoclonal antibody of FcRn decreases numbers of CD8+ T cells and the expression of pro-inflammatory cytokines in the gut.
**FIG. 11** depicts that pharmacological inhibition of FcRn decreases commensal (antigen)-specific CD4+ T cell activation in IgG-dependent colits.
**FIG. 12** depicts that human FcRn functionally binds to mouse IgG containing immune complexes.
**FIG. 13** depicts that human FcRn and human FcγR co-associated in antigen presenting cells.
**FIG. 14** depicts that hFcγR2a₁₃₁HIS exhibits stronger binding than hFcγR2a₁₃₁ARG across numerous IgG isotypes.

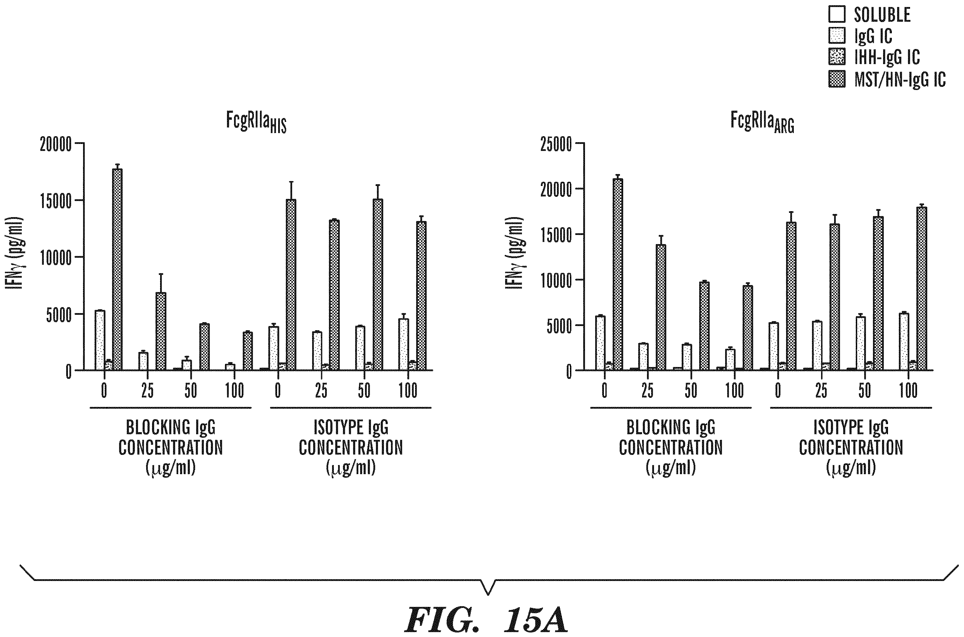

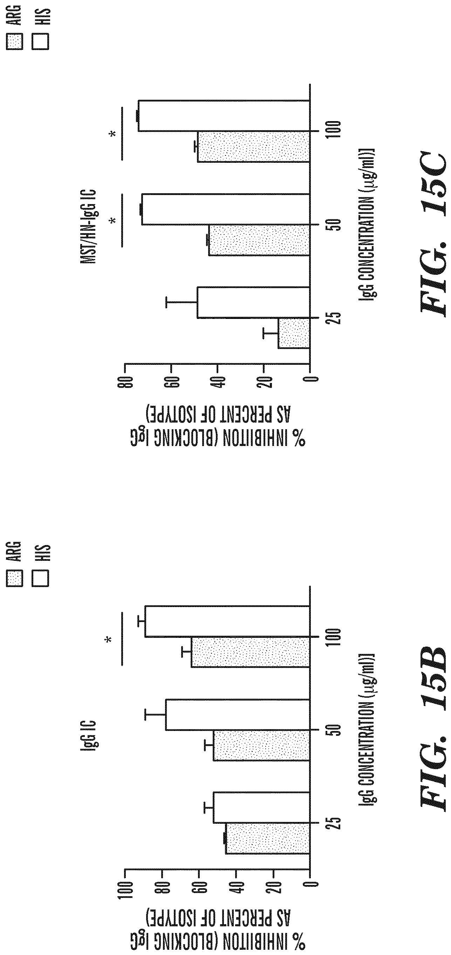

**FIGS. 15A-15C** depict that FcRn inhibition during in vitro cross-presentation assays leads to greater inhibition of CD8⁺ T cells in the lamina propria of hFcgRIIa₁₃₁HIS mice. Numerous polymorphisms of the hFcgRIIa gene exist. Transgenic mice expressing two of these allelic variants (hFcgrIIa₁₃₁HIS and hFcgrIIa₁₃₁ARG) on a murine FcgR deficient background were used as a source of DC for in vitro experiments. **FIG. 15A****.** FcRn blockade abrogates cross-presentation by DC from hFcgRIIa₁₃₁ mice. DC from hFcgRIIa₁₃₁ transgenic mice were incubated with IgG IC or a complex containing an enhanced FcRn-binding IgG variant (MST/HN-IgG IC) in combination with different doses of an FcRn blocking antibody (DVN24) or isotype control. DC were subsequently washed and incubated for 24 h with CD8+ T cells. Supernatant was collected and IFNg quantified via ELISA. (**FIGS**. **15B** and **15C**) Inhibition of FcRn led to a greater inhibition of T cell activation by DC taken from hFcgRIIa₁₃₁HIS as compared to hFcgRIIa₁₃₁ARG mice. Data from FIG. 15A was replotted as % inhibition (as compared to the corresponding isotype control). * *P* ≤ 0.05.

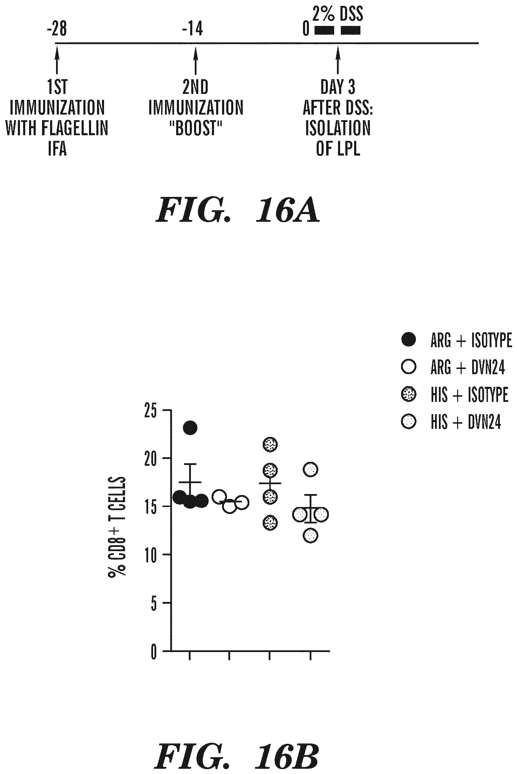

**FIGS. 16A-16B** depict that FcRn inhibition during colitis leads to greater changes in CD8+ T cells in the lamina propria of hFcgRIIa₁₃₁HIS mice. **FIG. 16A****.** Treatment regimen used for induction of IgG-dependent colitis. Mice were immunized with 10ug flagellin from S. typhimurium in incomplete Freund's adjuvant on days -28 and -14. On day 0, 2% DSS was added to the water and lamina propria content of T cells was assessed after 3 days of DSS treatment. **FIG. 16B****.** CD8+ T cells, which are known to be decreased in the lamina propria of *Fcgrt*^{*-*/*-*} mice, were evaluated by flow cytometry, gating on the CD3⁺CD8⁺ population of live cells in the lamina propria of the large intestine.

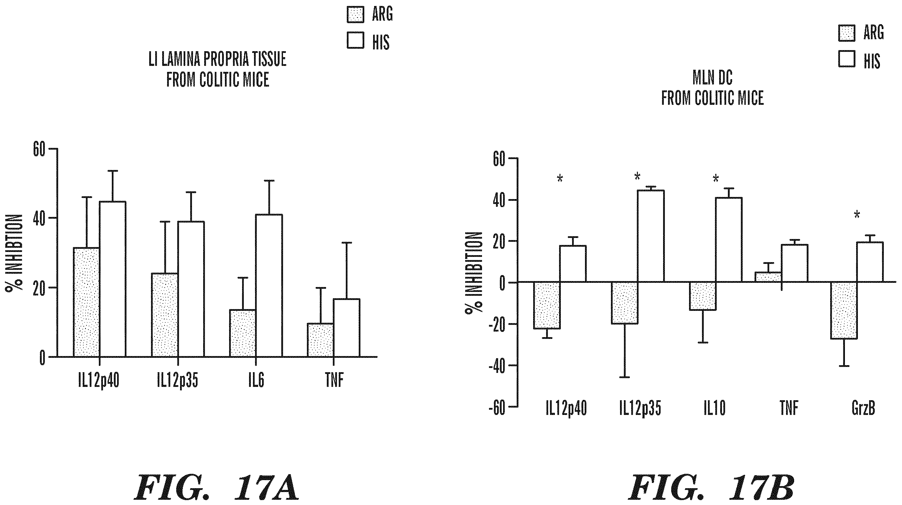

**FIGS. 17A-17B** depict that FcRn inhibition during colitis induces greater reduction in proinflammatory cytokine production in the intestinal tissue and MLN DC in hFegRIIa₁₃₁HIS mice. **FIG. 17A****.** % inhibition of whole tissue transcript levels for the indicated cytokines in mice treated with FcRn-blocking antibody as compared to isotype control treated mice. Mice were treated as in FIG. 16A and tissue transcripts were assessed after collection of intestinal tissue. **FIG. 17B****.** % inhibition of transcript levels of DC isolated from the mesenteric lymph node (MLN) for the indicated cytokines in mice treated with FcRn-blocking antibody as compared to isotype control treated mice. * *P* ≤ 0.05.

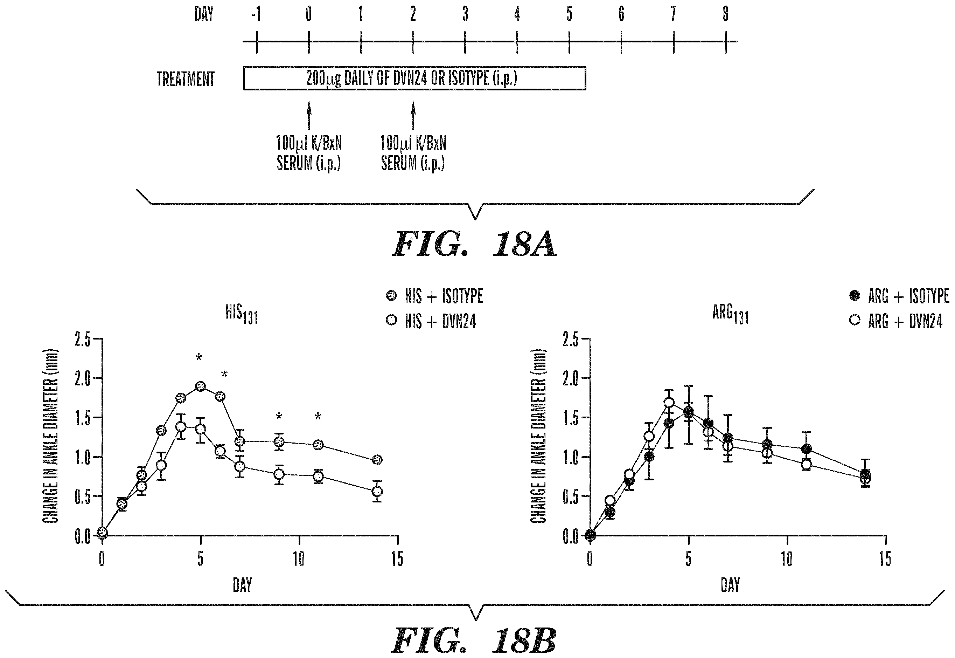

**FIGS. 18A-18B** depict that FcRn inhibition during arthritis induced by transfer of serum from K/BxN mice induces greater reduction in ankle thickness in hFcgRIIa₁₃₁HIS mice. **FIG. 18A****.** Treatment regiment for arthritis induction. Mice were treated with 200ug of FcRn-blocking antibody or isotype control on a daily basis from day -1 through day 5. On days 0 and 2, mice were administered 100ul of serum from K/BxN mice. Ankle thickness and inflammation were measured from day 0 through day 15. **FIG. 18B****.** Ankle thickness in hFcgRIIa₁₃₁HIS and hFcgRIIa₁₃₁ARG mice treated as in FIG. 15A. * P ≤ 0.05.

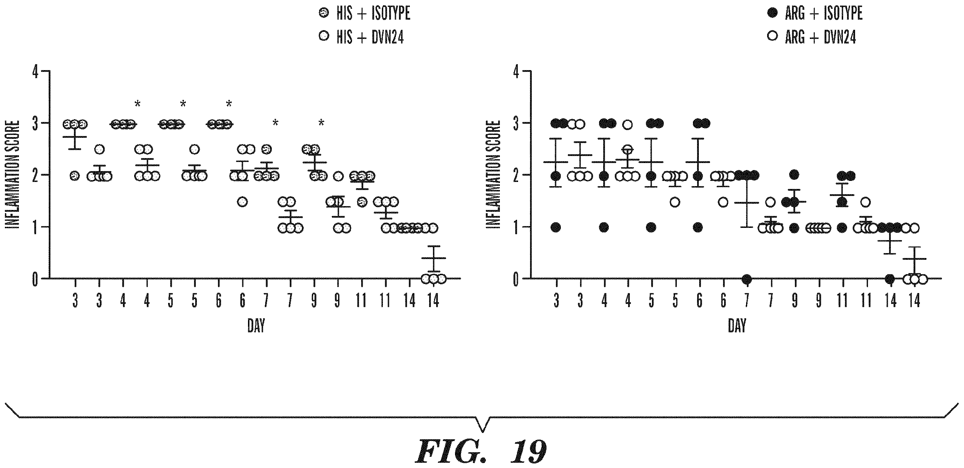

**FIG. 19** depicts that FcRn inhibition during arthritis induced by transfer of serum from K/BxN mice induces greater reduction in inflammation in hFcgRIIa₁₃₁HIS mice. Inflammation was scored based on the following criteria in mice treated as in FIG. 18A: 1 = mild swelling of the ankle insufficient to reverse the normal V shape of the foot (i.e., the foot at the base of the toes remains wider than the ankle and hindfoot); 2 = swelling sufficient to make the ankle and midfoot approximately equal in thickness to the forefoot; 3 = reversal of the normal V shape of the foot. * P ≤ 0.05.

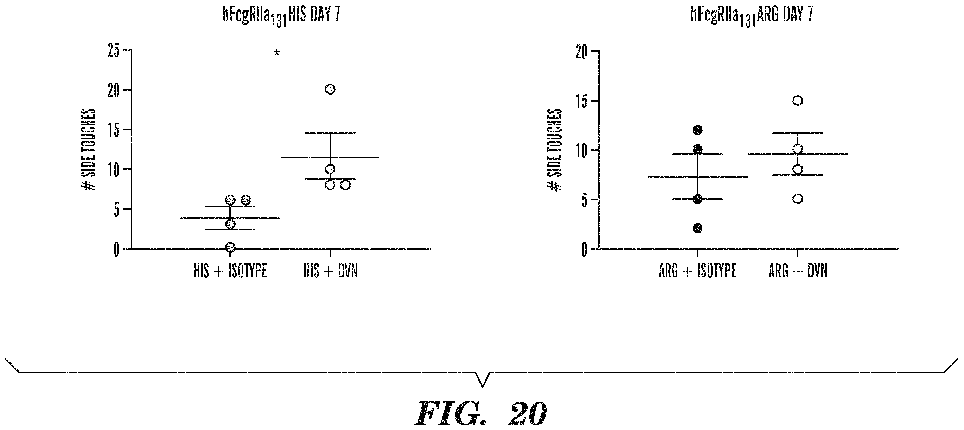

**FIG. 20** depicts that FcRn inhibition leads to preservation of mobility in mice chimeric for hFcgRIIa₁₃₁HIS but not hFcgRIIa₁₃₁ARG. Mobility was assessed in bone marrow chimeric mice (described in FIG. 20A) by placing the animal in a transparent beaker and assessing how many times the mouse lifted its forepaws and touched the side of the beaker in a 1 min time span. * P ≤ 0.05.

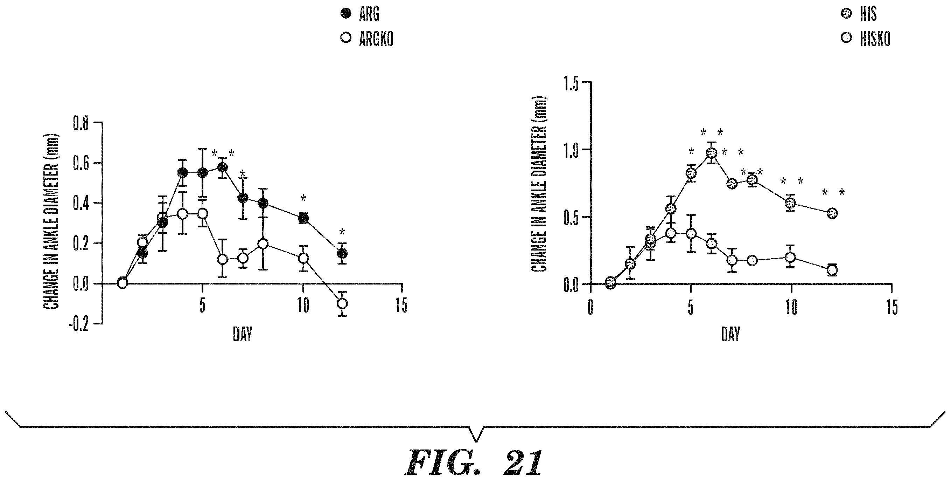

**FIG. 21** depicts that FcRn is required for the induction of arthritis by hFcgRIIa131-expressing hematopoietic cells. WT mice were irradiated with 2 x 600 rad and then reconstituted with bone marrow taken from hFcgRIIa₁₃₁HIS, hFcgRIIa₁₃₁HIS-mFcgrt^{-/-} (HISKO), hFcgRIIa₁₃₁ARG and hFcgRIIa₁₃₁ARG-mFcgrt^{-/-} (ARGKO) donors. 8 weeks after chimerization, mice were treated with K/BxN serum as in FIG. 18A. * P ≤ 0.05, ** P ≤ 0.01. Note the significantly decreased inflammation in the HISKO in comparison to the ARGKO mice relative to their littermate controls.

### DETAILED DESCRIPTION OF THE INVENTION

Unless defined otherwise, technical and scientific terms used herein have the same meaning as commonly understood by one of ordinary skill in the art to which this invention belongs. Allen et al., Remington: The Science and Practice of Pharmacy 22nd ed., Pharmaceutical Press (September 15, 2012); Hornyak et al., Introduction to Nanoscience and Nanotechnology, CRC Press (2008); Singleton and Sainsbury, Dictionary of Microbiology and Molecular Biology 3rd ed., revised ed., J. Wiley & Sons (New York, NY 2006); Smith, March's Advanced Organic Chemistry Reactions, Mechanisms and Structure 7th ed., J. Wiley & Sons (New York, NY 2013); Singleton, Dictionary of DNA and Genome Technology 3rd ed., Wiley-Blackwell (November 28, 2012); and Green and Sambrook, Molecular Cloning: A Laboratory Manual 4th ed., Cold Spring Harbor Laboratory Press (Cold Spring Harbor, NY 2012), provide one skilled in the art with a general guide to many of the terms used in the present application. For references on how to prepare antibodies, see Greenfield, Antibodies A Laboratory Manual 2nd ed., Cold Spring Harbor Press (Cold Spring Harbor NY, 2013); Köhler and Milstein, Derivation of specific antibody-producing tissue culture and tumor lines by cell fusion, Eur. J. Immunol. 1976 Jul, 6(7):511-9; Queen and Selick, *Humanized immunoglobulins*, U. S. Patent No. 5,585,089 (1996 Dec); and Riechmann et al., Reshaping human antibodies for therapy, Nature 1988 Mar 24, 332(6162):323-7.

One skilled in the art will recognize many methods and materials similar or equivalent to those described herein, which could be used in the practice of the present disclosure. Indeed, the present disclosure is in no way limited to the methods and materials described. For purposes of the present disclosure, the following terms are defined below.

As used herein, a "sample" or "biological sample" can refer to a solid, semi-solid or liquid sample, including, but not limited to, a tissue sample, a cellular sample, a cellular extract, plasma, serum, blood, cord blood, urine, body secretions from the nose, oropharynx, gastrointestinal tract, bile or genitourinary tract, tissue biopsies of any organ, a tissue fluid such as cerebrospinal, occular or joint fluids, or any combination thereof.

As used herein, an "immune-mediated disease" refers to any disease or disorder in which one or more components of the immune system, e.g., B cells, T cells, macrophages, antibodies, are involved in the initiation or pathogenesis of the disease or disorder, and/or there are elevated levels of one or more components of the immune system, and includes, but is not limited to, any one or more of monoclonal gammopathy of unknown significance (MGUS), inflammatory bowel disease, pemphigus or pemphigoid, myasthenia gravis, autoimmune hemolytic anemia, Kawasaki's disease, multiple myeloma, rheumatoid arthritis, systemic lupus erythematosus (SLE), cirrhosis of the liver, chronic hepatitis, chronic infection, multiple sclerosis (MS), macroglobulinemia, viral hepatitis, mononucleosis, kidney damage (nephrotic syndrome), celiac diseasae, allergic reactions, asthma, atopic dermatitis, cancer, idiopathic thrombocytopenic purpura (ITP), autoimmune thrombocytopenia, immune neutropenia, antiphospholipid syndrome, ANCA-associated disease, polymyositis, myasthenia gravis, diabetes, autoimmune thyroiditis, optic neuritis, Graves' opthalmopathy, Guillian-Barre Syndrome, chronic polyneuropathy, acute myeloid leukemia or a combination thereof.

As used herein, the term "inflammatory bowel disease" includes, for example, ulcerative colitis, Crohn's disease, collagenous colitis, lymphocytic colitis, ischaemic colitis, diversion colitis, Behçet's disease, indeterminate colitis or a combination thereof.

As used herein, in regard to a biological sample, the term "assaying" refers to any investigative or analytic method or procedure that can be used for qualitatively assessing or quantitatively measuring the presence or amount of any one of total IgG, a subclass of IgG, and/or antigen-specific IgG in a biological sample. For the aspects described herein, "assaying" encompasses any number of techniques and methods and includes, but is not limited to, DNA sequencing methods, including, but not limited to, Maxam-Gilbert sequencing, Chain-termination methods (Sanger Sequencing), Shotgun sequencing, Bridge PCR, massively parallel signature sequencing (MPSS), Polony sequencing, 454 pyrosequencing, Illumina (Solexa) sequencing, SOLiD sequencing, Ion Torrent semicondutor sequencing, DNA nanoball sequencing, Heliscope single molecule sequencing, Single molecule real time (SMRT) sequencing, Nanopore DNA sequencing, Tunnelling currents DN sequencing, sequencing by hybridization, sequencing with mass spectrometry, microfluidic Sanger sequencing, RNAP sequencing, or any combination thereof; techniques that can assess the amount of nucleic acid encoding IgG present in a sample, such as traditional "direct probe" methods such as Southern blots or *in situ* hybridization (*e.g.*, FISH and FISH plus SKY), "comparative probe" methods such as comparative genomic hybridization (CGH), (*e.g.*, cDNA-based or oligonucleotide-based CGH); methods of "quantitative" and "semi-quantitative" amplification, including fluorogenic quantitative PCR, ligase chain reaction (LCR), transcription amplification, self-sustained sequence replication, dot PCR, and linker adapter PCR, *etc.;* immunoassay techniques, including sandwich enzyme-linked immunoassays (ELISA), immunoabsorbent assays, immunoprecipitation assays, immunoblotting assays, radioimmunoassays (RIA), competitive binding assays, homogeneous assays, heterogeneous assays, etc.

As used herein, a "first therapy" refers to one or more of anti-inflammatories such as sulfasalazine, immunosuppressives, such as corticosteroids, immunomodulators, such as mercaptopurine, methotrexate or cyclosporine, biologic therapies, small molecule inhibitors of biologic pathways, such as kinase inhibitors; antibiotics, anti-diarrheal agents, laxatives, pain relievers, iron supplements, dietary modifications, Vitamin B-12 supplements, calcium supplements, vitamin D supplements or any combination thereof.

As used herein, a "second therapy" refers to anti-FcRn therapy, as the term is described herein.

The term "anti-FcRn therapy" refers to administration of an agent that blocks FcRn or an agent that reduces or inhibits expression of and/or signaling mediated by FcRn including, but limited to, any one or more of a peptide, protein (e.g., recombinant Fc portion of IgG or a biologically active portion thereof or a proteo-mimetic thereof), small molecule, nucleic acid (e.g., antisense RNA or siRNA specific for FcRn), aptamer, oligonucleotide, antibody or antigen-binding fragment thereof (e.g., monoclonal antibody or a fragment thereof, a polyclonal antibody or a fragment thereof, chimeric antibody, humanized antibody and single chain antibody), bispecific agent (for example, a bispecific antibody) or a combination thereof.

As used herein, the term "antibody" refers to an intact immunoglobulin or to a monoclonal or polyclonal antigen-binding fragment with the Fc (crystallizable fragment) region or FcRn binding fragment of the Fc region, referred to herein as the "Fc fragment" or "Fc domain". Antigen-binding fragments may be produced by recombinant DNA techniques or by enzymatic or chemical cleavage of intact antibodies. Antigen-binding fragments include, inter alia, Fab, Fab', F(ab')2, Fv, dAb, and complementarity determining region (CDR) fragments, single-chain antibodies (scFv), single domain antibodies, chimeric antibodies, diabodies, tetrabodies and other multimerized scFv moieties and polypeptides that contain at least a portion of an immunoglobulin that is sufficient to confer specific antigen binding to the polypeptide. The Fc domain includes portions of two heavy chains contributing to two or three classes of the antibody. The Fc domain may be produced by recombinant DNA techniques or by enzymatic (e.g. papain) cleavage or via chemical cleavage of intact antibodies.

The term "antibody fragment," as used herein, refer to a protein fragment that comprises only a portion of an intact antibody, generally including an antigen binding site of the intact antibody and thus retaining the ability to bind antigen. Examples of antibody fragments encompassed by the present definition include: (i) the Fab fragment, having VL, CL, VH and CH1 domains; (ii) the Fab' fragment, which is a Fab fragment having one or more cysteine residues at the C-terminus of the CH1 domain; (iii) the Fd fragment having VH and CH1 domains; (iv) the Fd' fragment having VH and CH1 domains and one or more cysteine residues at the C-terminus of the CH1 domain; (v) the Fv fragment having the VL and VH domains of a single arm of an antibody; (vi) the dAb fragment (Ward et al., Nature 341, 544-546 (1989)) which consists of a VH domain; (vii) isolated CDR regions; (viii) F(ab')2 fragments, a bivalent fragment including two Fab' fragments linked by a disulphide bridge at the hinge region; (ix) single chain antibody molecules (e.g., single chain Fv; scFv) (Bird et al., Science 242:423-426 (1988); and Huston et al., PNAS (USA) 85:5879-5883 (1988)); (x) "diabodies" with two antigen binding sites, comprising a heavy chain variable domain (VH) connected to a light chain variable domain (VL) in the same polypeptide chain (see, e.g., EP 404,097; WO 93/11161; and Hollinger et al., Proc. Natl. Acad. Sci. USA, 90:6444-6448 (1993)); (xi) "linear antibodies" comprising a pair of tandem Fd segments (VH-CH1-VH-CH1) which, together with complementary light chain polypeptides, form a pair of antigen binding regions (Zapata et al. Protein Eng. 8(10):1057-1062 (1995); and U.S. Pat. No. 5,641,870).

As described herein, an "antigen" is a molecule that is bound by a binding site on a polypeptide agent, such as an antibody or antibody fragment thereof. Typically, antigens are bound by antibody ligands and are capable of raising an antibody response in vivo. An antigen can be a polypeptide, protein, nucleic acid, lipid or other molecule. In the case of conventional antibodies and fragments thereof, the antibody binding site as defined by the variable loops (L1, L2, L3 and HI, H2, H3) is capable of binding to the antigen. The term "antigenic determinant" refers to an epitope on the antigen recognized by an antigen-binding molecule, and more particularly, by the antigen-binding site of said molecule.

An "Fv" fragment is an antibody fragment which contains a complete antigen recognition and binding site. This region consists of a dimer of one heavy and one light chain variable domain in tight association, which can be covalent in nature, for example in scFv. It is in this configuration that the three CDRs of each variable domain interact to define an antigen binding site on the surface of the VH-VL dimer. Collectively, the six CDRs or a subset thereof confer antigen binding specificity to the antibody. However, even a single variable domain (or half of an Fv comprising only three CDRs specific for an antigen) has the ability to recognize and bind antigen, although usually at a lower affinity than the entire binding site.

As used herein, an "epitope" can be formed both from contiguous amino acids, or noncontiguous amino acids juxtaposed by tertiary folding of a protein. Epitopes formed from contiguous amino acids are typically retained on exposure to denaturing solvents, whereas epitopes formed by tertiary folding are typically lost on treatment with denaturing solvents. An epitope typically includes at least 3, and more usually, at least 5, about 9, or about 8-10 amino acids in a unique spatial conformation. An "epitope" includes the unit of structure conventionally bound by an immunoglobulin V_{H}/V_{L} pair. Epitopes define the minimum binding site for an antibody, and thus represent the target of specificity of an antibody. In the case of a single domain antibody, an epitope represents the unit of structure bound by a variable domain in isolation. The terms "antigenic determinant" and "epitope" can also be used interchangeably herein. In various embodiments, an epitope may be protein, peptide, nucleic acid, lipid, other molecules or combinations thereof.

The term "monoclonal antibody," as used herein, refers to an antibody that is part of a preparation of antibody molecules of single molecular composition. A population of monoclonal antibodies has a single binding specificity and affinity for a particular epitope, and the antibodies are identical except for possible naturally occurring mutations that can be present in minor amounts. Accordingly, the term "monoclonal antibody" refers to an antibody that is derived from a single clone, including any eukaryotic, prokaryotic, or phage clone, and not the method by which it is produced. Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, 2nd ed., (Cold Spring Harbor Laboratory Press, Cold Spring Harbor, 1988); Hammerling, et al. eds., in: Monoclonal Antibodies and T-Cell Hybridomas "In Research Monographs in Immunology, vol. 3 (J. L. Turk, General Editor) (Elsevier, N.Y., 1981), Kohler et al., Nature 256:495 (1975); can be made by recombinant DNA methods (see, e.g., U.S. Pat. No. 4,816,567); or can also be isolated from phage antibody libraries using the techniques described in Clackson et al., Nature 352:624-628 (1991) or Marks et al., J. Mol. Biol. 222:581-597 (1991), for example. The modifier "monoclonal" is not to be construed as requiring production of the antibody by any particular method.

As used herein, "selectively binds" or "specifically binds" refers to the ability of an antibody or antibody fragment thereof described herein to bind to a target, such as a molecule present on the cell-surface, with a KD 10⁻⁵ M (10000 nM) or less, e.g., 10⁻⁶ M, 10⁻⁷ M, 10⁻⁸ M, 10⁻⁹ M, 10⁻¹⁰ M, 10⁻¹¹ M, 10⁻¹² M, or less, but not to another molecule with similar affinity. Specific binding can be influenced by, for example, the affinity and avidity of the polypeptide agent and the concentration of polypeptide agent. The person of ordinary skill in the art can determine appropriate conditions under which the polypeptide agents described herein selectively bind the targets using any suitable methods, such as titration of a polypeptide agent in a suitable cell binding assay.

"Subject" or "individual" or "animal" or "patient" or "mammal," is meant any subject, particularly a mammalian subject, for whom diagnosis, prognosis, or therapy is desired. In some embodiments, the subject has inflammatory bowel disease. In some embodiments, the subject had inflammatory bowel disease at some point in the subject's lifetime. In some embodiments, the subject has an autoimmune disease.

"Mammal" as used herein refers to any member of the class *Mammalia*, including, without limitation, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; rodents such as mice, rats, hamsters and guinea pigs; and so on. In certain embodiments, the mammal is a human subject. The term does not denote a particular age or sex.

As used herein, the term "target" refers to a biological molecule (e.g., peptide, polypeptide, protein, lipid, carbohydrate) to which a polypeptide domain which has a binding site can selectively bind. The target can be, for example, an intracellular target (e.g., an intracellular protein target) or a cell surface target (e.g., a membrane protein, a receptor protein). Preferably, a target is a cell surface target, such as a cell surface protein.

As used herein, the terms "treat," "treatment," "treating," or "amelioration" refer to therapeutic treatments, wherein the object is to reverse, alleviate, ameliorate, inhibit, slow down or stop the progression or severity of a condition associated with, a disease or disorder. The term "treating" includes reducing or alleviating at least one adverse effect or symptom of a condition, disease or disorder, such as an autoimmune disease, a chronic infection or a cancer. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation or at least slowing of progress or worsening of symptoms that would be expected in absence of treatment. Beneficial or desired clinical results include, but are not limited to, alleviation of one or more symptom(s), diminishment of extent of disease, stabilized (i.e., not worsening) state of disease, delay or slowing of disease progression, amelioration or palliation of the disease state, and remission (whether partial or total), whether detectable or undetectable. The term "treatment" of a disease also includes providing relief from the symptoms or side-effects of the disease (including palliative treatment).

As used herein, the terms "administering," and "introducing" are used interchangeably and refer to the placement of an agent, such as an agent that blocks or inhibits FcRn, into a subject by a method or route which results in at least partial localization of such agents at a desired site, such as a site of inflammation, such that a desired effect(s) is produced, and includes but is not limited to, injection, infusion, instillation, and inhalation administration.

The term "effective amount" as used herein refers to the amount of an agent for blocking or inhibiting FcRn needed to alleviate at least one or more symptom of the disease or disorder, and relates to a sufficient amount of pharmacological composition to provide the desired effect, for example, treating IBD. The term "therapeutically effective amount" therefore refers to an amount of an agent for blocking FcRn using the methods as disclosed herein, that is sufficient to effect a particular effect when administered to a typical subject. An effective amount as used herein would also include an amount sufficient to delay the development of a symptom of the disease, alter the course of a symptom of disease (for example but not limited to, slow the progression of a symptom of the disease), or reverse a symptom of disease. Thus, it is not possible to specify the exact "effective amount". However, for any given case, an appropriate "effective amount" can be determined by one of ordinary skill in the art using only routine experimentation.

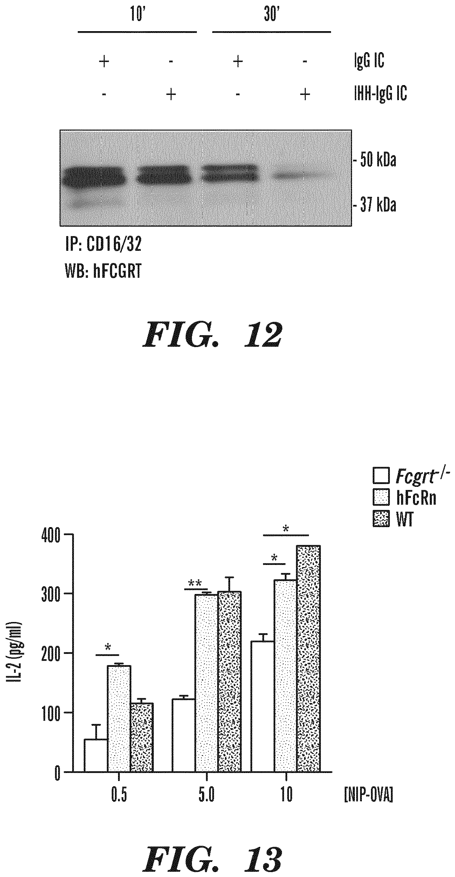

FcRn is a neonatal Fc receptor. It is similar in structure to major histocompatibility complex I (MHC I). Human FcRn is very stringent regarding its specificity and binds human Fc, but not mouse, rat, bovine, or sheep Fc. The FcRn can bind to two sites of the IgG (Sanchez et al. Biochemistry 1999; 38(29):9471-6; Schuck et al., Mol Immunol. 1999 36(15-16):1117-25; West A.P. and Bjorkman, 2000 Biochemistry 2000 39(32):9698-708). Although mouse IgGs do not bind efficiently to human FcRn and therefore have a short half-life in humans (Frodin et al., 1990), mouse IgG as an immune complex is capable of binding human FcRn and inducing signaling (see, e.g., FIG. 12). In contrast, mouse FcRn binds IgG from every species analyzed (Ober et al., Int Immunol. 2001 13(12):1551-9).

Most serum proteins have a short serum half-life (about 1-2 days). However, two types of serum proteins, namely albumin and antibodies of the IgG class, have greatly extended serum half-lives. For example, most subclasses of IgG have a half-life of about 10-20 days in humans. The Fc region of IgG is required for this extension of half-life. Thus, truncated IgG polypeptides carrying only the Fc region, and potentially also proteins carrying a short FcRn binding peptide sequence (FcBP) (Sockolosky et al. Proc Natl Acad Sci U S A 2012, 109, 16095-100), also show such extended serum half-life. Moreover, when the Fc region is fused with a fusion partner (e.g., a biologically active protein), this Fc fusion protein shows an extended serum half-life due to its interaction with FcRn.

FcgammaR2a (FcγR2a) is a classical Fc receptor for IgG that has previously been demonstrated to exhibit a low affinity for IgG on its own, and thus mainly associated with interactions with IgG immune complexes, *i.e.*, IgG bound to an antigen. FcγR2a exhibits allelism at amino acid residue 131, such that humans express either a His (also known as His 167 according to NM_001136219.1) or Arg residue (also known as Arg167 according to NM_001136219.1). The allele frequency differs among Caucasians (25% R/R, 50% R/H, 25% H/H) and non-Caucasians, such as Asians (5 % R/R, 35 % R/H, 60% H/H), with the His variant exhibiting higher affinity for human IgG2, even when a monomer.

The His131 (HIS131) variant of FcγR2a is a genetic risk allele for several immune mediated diseases, such as Kawasaki's disease and inflammatory bowel disease in both Caucasians and non-Caucasians, such that individuals with this allele exhibit a higher risk for these diseases. The inventors have discovered that in addition to exhibiting a higher affinity for IgG2, the His variant of FcγR2a exhibits stronger signaling when bound to IgG2 and IgG1 immune complexes, and that this is associated with increased production of inflammatory cytokines and enhancement of both MHC class I and MHC class II presentation of immune complex-bound antigens. Among the cytokines that are increased by the FcγR2a His131 (also known as His167 according to NM_001136219.1) allele is IL-12.

The increased signaling and antigen presentation and, thus, activation of T cells which is associated with excessive immune complex binding and signaling by the His allele described herein is abrogated by blockade or elimination of FcRn expression by antigen presenting cells, as demonstrated herein. This indicates that any individual with the FcγR2a-His131 (also known as His167 according to NM_001136219.1) allele will exhibit much greater IgG driven inflammation, and that blockade of inflammation with FcRn-targeted therapies can eliminate this inflammation. Thus, patients with an immune mediated disease that is characterized by elevated IgG levels can be selected for enhanced responses to anti-FcRn therapy based upon their FcγR2a allele usage. In some embodiments, subjects with elevated levels of anti-bacterial IgG and having the FcγR2a-His131 allele (also known as His167 according to NM_001136219.1) would be predicted to respond to FcRn-targeted therapies, as described herein.

Accordingly, described herein, in some aspects, are assays for using elevated IgG levels for selecting patients for therapy. Provided herein, in one aspect, is an assay comprising providing a biological sample from a subject having an immune-mediated disease or disorder that is characterized by increased levels of IgG (for example, inflammatory bowel disease) and selecting the subject for a first therapy. In some embodiments, the subject is human. In some embodiments, the subject is administered a composition comprising the first therapy. The subject being administered the composition comprising the first therapy may optionally be administered a composition comprising a second therapy either simultaneously or sequentially.

Also described herein, in some aspects, are assays for using elevated IgG levels and FcγR2a allele usage for selecting patients for anti-FcRn therapies. Provided herein is an assay comprising providing a biological sample from a subject having an immune-mediated disease that is characterized by increased levels of total IgG, specific IgG isotypes, or antigen specific IgG (e.g. anti-desmoglein, anti-insulin, anti-flagellin), assaying the sample to determine the allele of FcγR2a expressed by the subject, and selecting the subject for a first therapy if the subject expresses the Arg131 (also known as Arg167 according to NM_001136219.1) allele of FcγR2a, and selecting the subject for a second therapy if the subject expresses a His131 (also known as His167 allele according to NM_001136219.1) allele of FcγR2a. In some embodiments, the subject is human. In some embodiments, the subject is administered a composition comprising the first therapy. The subject being administered the composition comprising the first therapy may also be administered a composition comprising the second therapy either simultaneously or sequentially.

Further described herein is an assay for selecting a therapy for a subject suspected of having or having an immune-mediated disease, and optionally administering the therapy. The assay includes providing a biological sample from a subject having or suspected of having an immune-mediated disease, assaying the sample to determine the allele of FcγR2a, and selecting the subject for a first therapy if the subject expresses a Arg131 allele of FcγR2a, and selecting a second therapy if the subject expresses the His131 allele of FcγR2a. In some embodiments, the subject prescribed the first therapy may also be prescribed the second therapy.

In various embodiments of the assays described herein, the assays further comprise prescribing a first therapy or a combination of a first and second therapy to the subject, if the subject expresses the Arg131 allele of FcγR2a and increased IgG levels, or prescribing a second therapy if the subject expresses the His131 allele of FcγR2a (and optionally increased IgG levels). In some embodiments, the subject has or is suspected of having an immune-mediated disease.

Also described herein is an assay for selecting a therapy for a subject having a disease-state, and, optionally, administering the therapy. The assay comprises providing a biological sample from a subject having the disease-state, assaying the sample to determine the allele of FcγR2a expressed by the subject, and selecting the subject for a first therapy and optionally a second therapy, if the subject expresses a Arg131 allele of FcγR2a, and selecting a second therapy if the subject expresses the His131 allele of FcγR2a. In various embodiments, the disease-state is any disease-state that is associated with the expression of the His131 allele of FcγR2a. In some embodiments, the disease-state is any one or more of diseases of the gonads, diseases of the central nervous system, diseases of the eye, and diseases of the brain, or a combination thereof.

Further provided herein, in some aspects, are methods for treating immune-mediated diseases, inhibiting immune-mediated diseases, and/or reducing the severity of immune-mediated diseases in a subject in need thereof. The methods comprise providing a biological sample from a subject having an immune-mediated disease or disorder that is characterized by increased levels of total, isotype or antigen specific IgG, assaying the sample to determine the allele of FcγR2a expressed by the subject, selecting the subject for first therapy if the subject expresses the Arg131 (also known as Arg167 according to NM_001136219.1) allele of FcγR2a and selecting the subject for a second therapy if the subject expresses a His131 (also known as His167 allele according to NM_001136219.1) allele of FcγR2a and administering an effective amount of a composition comprising a first therapy and optionally a second therapy if the subject expresses the Arg131 allele of FcγR2a or administering an effective amount of a composition comprising a second therapy if the subject expresses the Arg131 allele of FcγR2a so as to treat, inhibit or reduce the severity of immune-mediated disease in the subject.

Further, provided herein are methods for treating immune-mediated diseases, inhibiting immune-mediated diseases and/or reducing the severity of immune-mediated diseases in a subject in need thereof. The methods comprise providing a biological sample from a subject having or suspected of having an immune-mediated disease, assaying the sample to determine the allele of FcγR2a and selecting the subject for first therapy if the subject expresses a Arg131 allele of FcγR2a and selecting a second therapy if the subject expresses the His131 allele of FcγR2a and administering an effective amount of a composition comprising a first therapy and optionally a second therapy if the subject expresses the Arg131 allele of FcγR2a or administering an effective amount of a composition comprising a second therapy if the subject expresses the Arg131 allele of FcγR2a so as to treat, inhibit or reduce the severity of immune-mediated disease in the subject. The His131 allele is also known as His167 allele according to NM_001136219.1. The Arg131 allele is also known as Arg167 allele according to NM_001136219.1.

Also provided herein is an assay for assessing the responsiveness of a subject having immune-mediated disease that is undergoing the second therapy, to the second therapy. In various embodiments, the second therapy is anti-FcRn therapy, as described herein. The assay comprises providing a biological sample from a subject having an immune-mediated disease that is undergoing anti-FcRn therapy and assaying the sample to determine the levels of IgG in the sample relative to a reference value. In some embodiments, in subjects undergoing the anti-FcRn therapy, a decrease in the total, isotype and/or antigen specific IgG levels relative to the reference value may be indicative of a subject being responsive to the anti-FcRn therapy. Alternately, absence of change in IgG levels or an increase in the IgG levels in the subject undergoing the anti-FcRn therapy may be indicative of the subject being non-responsive to the anti-FcRn therapy.

In some embodiments, the immune-mediated disease includes, but is not limited to, any one or more of monoclonal gammopathy of unknown significance (MGUS), inflammatory bowel disease, pemphigus or pemphigoid, myasthenia gravis, autoimmune hemolytic anemia, Kawasaki's disease, multiple myeloma, rheumatoid arthritis, systemic lupus erythematosus (SLE), cirrhosis of the liver, chronic hepatitis, chronic infection, multiple sclerosis (MS), macroglobulinemia, viral hepatitis, mononucleosis, kidney damage (nephrotic syndrome), celiac diseasae, allergic reactions, asthma, atopic dermatitis, cancer, idiopathic thrombocytopenic purpura (ITP), autoimmune thrombocytopenia, immune neutropenia, antiphospholipid syndrome, ANCA-associated disease, polymyositis, myasthenia gravis, diabetes, autoimmune thyroiditis, optic neuritis, Graves' opthalmopathy, Guillian-Barre Syndrome, chronic polyneuropathy, acute myeloid leukemia or a combination thereof

In some embodiments, the subject has undergone or is undergoing treatment for inflammatory bowel disease. In some aspects, the subject has not undergone or is not currently undergoing treatment for inflammatory bowel disease. In various embodiments, IBD is ulcerative colitis, Crohn's disease, collagenous colitis, lymphocytic colitis, ischaemic colitis, diversion colitis, Behçet's disease, indeterminate colitis or a combination thereof.

In an embodiment, the subject selected for second therapy expresses a His131 allele of FcγR2a. The subject may be heterozygous for the His131 allele of FcγR2a or homozygous for the His131 allele of FcγR2a. The His131 allele is also known as His167 allele according to NM_001136219.1.

In various embodiments, the first therapy is any one or more of anti-inflammatories such as sulfasalazine, immunosuppressives, such as corticosteroids, immunomodulators, such as mercaptopurine, methotrexate or cyclosporine, biologic therapies, or small molecule inhibitors of biologic pathways, such as kinase inhibitors.

In some aspects, the first therapy in subjects with IBD is any one or more of antiinflammatory agents, immune-suppressing agent, antibiotics, anti-diarrheal agents, laxatives, pain relievers, iron supplements, dietary modifications, Vitamin B-12 supplements, calcium supplements, vitamin D supplements or a combination thereof.

In various embodiments, the second therapy comprises anti-FcRn therapy as described herein. The anti-FcRn therapy may comprise an agent that blocks FcRn or an agent that reduces or inhibits expression of FcRn. In some embodiments, agents that blocks FcRn may be any one or more of a peptide, protein, small molecule, nucleic acid, aptamer, oligonucleotide, antibody or a combination thereof. The nucleic acid may be a siRNA specific to FcRn.

In various embodiments, the antibody for use in the anti-FcRn therapy specifically recognizes and binds FcRn and is selected from the group consisting of a monoclonal antibody or an antigen-binding fragment thereof, a polyclonal antibody or an antigen-binding fragment thereof, chimeric antibody, humanized antibody and single chain antibody. In various embodiments, the antibody specific to FcRn inhibits the signaling medicated by FcRn.

In some embodiments, the agent that blocks FcRn is a bispecific agent (for example, a bispecific antibody) comprising binding sites for Fcγ receptors and FcRn. In some embodiments, an agent targets FcRn and a second, different agent targets the Fcγ receptors and the two agents may be administered simultaneously or sequentially.

In some embodiments, the agent that blocks FcRn is a bispecific agent (for example, a bispecific antibody) comprising binding sites for IgG and Fcγ receptors.

In some embodiments, the agent that blocks FcRn is a bispecific agent (for example, a bispecific antibody) comprising binding sites for IgG and FcRn.

In some embodiments, the agent that blocks FcRn is a recombinant Fc portion of IgG or a biologically active portion thereof or a proteo-mimetic thereof. The Fc portion of IgG or a biologically active portion thereof may be mammalian. The Fc portion of IgG or a biologically active portion thereof may be human.

In some embodiments, the agent that blocks FcRn is a SYK inhibitor. As used herein, a "SYK inhibitor" inhibits or reduces SYK-mediated signaling.

Also provided herein is a system for determining responsiveness of a subject to anti-FcRn therapy wherein a sample is obtained from a subject diagnosed with an immune-mediated disease. The system comprises a sample analyzer configured to produce a signal for mRNA encoding IgG isotypes present in the sample obtained from the subject and a computer sub-system programmed to calculate, based on the IgG mRNA isotypes whether the signal is greater than or not greater than a reference value.

Further provided herein is a system for determining responsiveness of a subject to anti-FcRn therapy wherein a sample is obtained from a subject diagnosed with an immune-mediated disease. The system includes a sample analyzer configured to produce a signal for IgG isotypes present in the sample obtained from the subject and a computer sub-system programmed to calculate, based on the IgG isotypes whether the signal is greater than or not greater than a reference value.

In various embodiments, the computer sub-system is programmed to compare the mRNA or protein to determine a likelihood of responsiveness of the subject to anti-FcRn therapy based on an algorithm that classifies the subject as responsive if IgG level is decreased and as not responsive to anti-FcRn therapy if IgG isotype levels are not decreased. In some embodiments, the reference value is the mean or median IgG isotype expression levels from a population of subjects that do not have immune-mediated disease. In some embodiments, the reference value is the mean or median IgG isotype expression levels from a population of subjects that have immune-mediated disease and respond to anti-FcRn therapy.

Also provided herein is a diagnostic kit for detecting the likelihood of a subject expressing the His131 FcγR2a allele (for example, subject with immune-mediated disease) responding to anti-FcRn therapy. The kit includes, for example, about 10 probes comprising a combination of detectable labeled probes or primers for all IgG or specific IgG isotypes and a computer program product described herein. The kit can include more than 10 probes or less than 10 probes.

Also provided herein is a diagnostic kit for detecting the likelihood of a subject expressing the His131 FcγR2a allele (for example, subject with immune-mediated disease) responding to anti-FcRn therapy. The kit includes, for example, about 10 probes comprising a combination of detectable labeled antibodies or siRNAs that bind total IgG or specific IgG isotypes and a computer program product described. The kit can include more than 10 probes or less than 10 probes.

In some embodiments, the biological sample is solid, semi-solid or liquid. In an embodiment, the sample is tissue (Baker K, et al., Immunity 2013 39(6):1095-107). In various embodiments, the sample is sample is serum or blood, body secretions from the nose, oropharynx, gastrointestinal tract, bile or genitourinary tract, tissue biopsies of any organ, a tissue fluid such as cerebrospinal, occular or joint or a combination thereof.

### Analysis of FcγR2a allele and IgG expression

In various embodiments, the assays, methods and systems described herein comprise analyzing the allele of FcγR2a in a biological sample and/or the expression level of total IgG, levels of specific isotypes of IgG and/or antigen specific IgG levels in a biological sample.

In various embodiments of the assays and methods described herein, analyzing the sample includes detecting the allele of FcγR2a with an FcγR2a allele specific antibody (for example, an antibody that is specific to an epitope comprising the His131 allele of FcγR2a or an antibody that is specific to an epitope comprising the Arg131 allele of FcγR2a (for example MAb41H16) (Gosselin, EJ, Brown, MF, Anderson, CL, Zipf, TF, Guyre, PM. The monoclonal antibody 41H16 detects the Leu 4 responder form of human FcgRII. J Immunol 1990. 144:1817-1822). In some embodiments of the assays and methods described herein, analyzing the sample includes detecting the level of total IgG, isotypes of IgG or antigen specific IgG levels. In various embodiments, the antibodies are any one or more of a monoclonal antibody or fragment thereof, a polyclonal antibody or a fragment thereof, chimeric antibodies, humanized antibodies, human antibodies, and a single chain antibody. In an embodiment, the antibody is a monoclonal antibody.

As will be apparent to one skilled in the art, DNA sequencing methods may be used to analyze the allele of FcγR2a in the sample. DNA sequencing methods include but are not limited to Maxam-Gilbert sequencing, Chain-termination methods (Sanger Sequencing), Shotgun sequencing, Bridge PCR, massively parallel signature sequencing (MPSS), Polony sequencing, 454 pyrosequencing, Illumina (Solexa) sequencing, SOLiD sequencing, Ion Torrent semicondutor sequencing, DNA nanoball sequencing, Heliscope single molecule sequencing, Single molecule real time (SMRT) sequencing, Nanopore DNA sequencing, Tunnelling currents DN sequencing, sequencing by hybridization, sequencing with mass spectrometry, microfluidic Sanger sequencing, RNAP sequencing, or any combination thereof. In addition, as new sequencing methods are developed, these can be applied to the analysis of the allele of FcγR2a in a biological sample, as understood by one of ordinary skill in the art.

In some embodiments of the assays and methods described herein, analyzing the sample includes detecting the mRNA that encodes a specific allele of FcγR2a with a polynucleotide capable of hybridizing with mRNA specific for the allele of FcγR2a under stringent hybridization conditions. In one embodiment, the polynucleotide hybridizes with the His131 allele of FcγR2a but not with the Arg131 allele of FcγR2a. In another embodiment, the polynucleotide hybridizes with the Arg131 allele of FcγR2a but not with the His131 allele of FcγR2a.

In some embodiments of the assays and methods described herein, analyzing the sample includes measuring the levels of mRNA that encode isotypes of IgG present in the sample with a polynucleotide capable of hybridizing with mRNA specific for each IgG isotype under stringent hybridization conditions.

Techniques that may be used to assess the amount of nucleic acid encoding IgG, present in the sample include but are not limited to *in situ* hybridization (*e.g.*, Angerer (1987) Meth. Enzymol 152: 649). Preferred hybridization-based assays include, but are not limited to, traditional "direct probe" methods such as Southern blots or *in situ* hybridization (*e.g.*, FISH and FISH plus SKY), and "comparative probe" methods such as comparative genomic hybridization (CGH), *e.g.,* cDNA-based or oligonucleotide-based CGH. The methods can be used in a wide variety of formats including, but not limited to, substrate (e.g. membrane or glass) bound methods or array-based approaches. Probes that may be used for nucleic acid analysis are typically labeled, *e.g.*, with radioisotopes or fluorescent reporters. Preferred probes are sufficiently long so as to specifically hybridize with the target nucleic acid(s) under stringent conditions. The preferred size range is from about 200 bases to about 1000 bases. Hybridization protocols suitable for use with the methods of the present disclosure are described, *e.g.,* in Albertson (1984) EMBO J. 3: 1227-1234; Pinkel (1988) Proc. Natl. Acad. Sci. USA 85: 9138-9142; EPO Pub. No. 430,402; Methods in Molecular Biology, Vol. 33: In situ Hybridization Protocols, Choo, ed., Humana Press, Totowa, N.J. (1994), Pinkel, et al. (1998) Nature Genetics 20: 207-211, and/or Kallioniemi (1992) Proc. Natl Acad Sci USA 89:5321-5325 (1992).

Methods of "quantitative" amplification are well known to those of skill in the art. For example, quantitative PCR involves simultaneously co-amplifying a known quantity of a control sequence using the same primers. This provides an internal standard that may be used to calibrate the PCR reaction. Detailed protocols for quantitative PCR are provided in Innis, et al. (1990) PCR Protocols, A Guide to Methods and Applications, Academic Press, Inc. N.Y.). Measurement of DNA copy number at microsatellite loci using quantitative PCR anlaysis is described in Ginzonger, et al. (2000) Cancer Research 60:5405-5409. The known nucleic acid sequence for the genes is sufficient to enable one of skill in the art to routinely select primers to amplify any portion of the gene. Fluorogenic quantitative PCR may also be used in the methods of the present disclsoure. In fluorogenic quantitative PCR, quantitation is based on amount of fluorescence signals, *e.g*., TaqMan and sybr green.

Other suitable amplification methods include, but are not limited to, ligase chain reaction (LCR) (see Wu and Wallace (1989) Genomics 4: 560, Landegren, et al. (1988) Science 241:1077, and Barringer et al. (1990) Gene 89: 117), transcription amplification (Kwoh, et al. (1989) Proc. Natl. Acad. Sci. USA 86: 1173), self-sustained sequence replication (Guatelli, et al. (1990) Proc. Nat. Acad. Sci. USA 87: 1874), dot PCR, and linker adapter PCR, *etc.*

A two-tailed student t-test with unequal variation may be used to measure the differences between the patient's expression of total IgG, IgG isotypes and/or antigen specific IgG levels and a normal sample, or the patient's own sample (matched control), or a reference generated by computer algorithm pooling many control samples. A significant difference may be achieved where the p value is equal to or less than 0.05. IgG mRNA expression may also be used to determine patient's prognosis and response to anti-FcRn therapy, where IgG mRNA expression is separated into two groups: those with high IgG expression and those with low or no detectable IgG expression. The groups may be separated by the median IgG expression and plotted over time with a Kaplan-Meier curve.

Suitable methods for assaying the expression level of total IgG or isotypes of IgG include, but are not limited to, using DNA sequencing, comparative genomic hybridization (CGH), array CGH (aCGH), SNP analysis, mRNA expression assay, RT-PCR, real-time PCR, or a combination thereof. In various embodiments, the assay to detect the nucleic acid encoding, or protein levels of, total IgG or isotypes of IgG , is any one or more of Northern blot analysis, Southern blot analysis, reverse transcription-polymerase chain reaction (RT-PCR), polymerase chain reaction (PCR), enzyme-linked immunosorbent assay (ELISA), radio-immuno assay (RIA), sodium dodecyl sulfate polyacrylamide gel electrophoresis (SDS-PAGE), Western blot analysis or a combination thereof.

Antibodies, both polyclonal and monoclonal, can be produced by a skilled artisan either by themselves using well known methods or they can be manufactured by service providers who specialize in making antibodies based on known protein sequences. In the present disclosure, the protein sequences are known and thus production of antibodies against them is a matter of routine experimentation.

For example, production of monoclonal antibodies can be performed using the traditional hybridoma method by first immunizing mice with an antigen which may be an isolated protein of choice or fragment thereof (for example, fragment comprising the His131 allele of FcγR2a or a fragment comprising the Arg131 allele of FcγR2a or IgG or a fragment thereof or a variant thereof) and making hybridoma cell lines that each produce a specific monoclonal antibody. The antibodies secreted by the different clones are then assayed for their ability to bind to the antigen using, e.g., ELISA or Antigen Microarray Assay, or immuno-dot blot techniques. The antibodies that are most specific for the detection of the protein of interest can be selected using routine methods and using the antigen used for immunization and other antigens as controls. The antibody that most specifically detects the desired antigen and protein and no other antigens or proteins are selected for the assays and methods described herein.

The best clones can then be grown indefinitely in a suitable cell culture medium. They can also be injected into mice (in the peritoneal cavity, surrounding the gut) where they produce an antibody-rich ascites fluid from which the antibodies can be isolated and purified. The antibodies can be purified using techniques that are well known to one of ordinary skill in the art.

In the methods and assays of the present disclosure, the presence of a specific allele of FcγR2a or the presence and/or level of IgG is determined using antibodies specific for the FcγR2a allele or for IgG and detecting immunospecific binding of each antibody to its respective cognate marker.

Any suitable immunoassay method may be utilized, including those which are commercially available. Extensive discussion of the known immunoassay techniques is not required here, since these are known to those of skill in the art. Typical suitable immunoassay techniques include sandwich enzyme-linked immunoassays (ELISA), radioimmunoassays (RIA), competitive binding assays, homogeneous assays, heterogeneous assays, etc. Various known immunoassay methods are reviewed, e.g., in Methods in Enzymology, 70, pp. 30-70 and 166-198 (1980).

In the assays of the present disclosure, "sandwich-type" assay formats can be used. Some examples of such sandwich-type assays are described in by U.S. Pat. No. 4,168,146 to Grubb, et al. and U.S. Pat. No. 4,366,241 to Tom, et al. An alternative technique is the "competitive-type" assay. In a competitive assay, the labeled probe is generally conjugated with a molecule that is identical to, or an analog of, the analyte. Thus, the labeled probe competes with the analyte of interest for the available receptive material. Competitive assays are typically used for detection of analytes such as haptens, each hapten being monovalent and capable of binding only one antibody molecule. Examples of competitive immunoassay devices are described in U.S. Pat. No. 4,235,601 to Deutsch, et al., U.S. Pat. No. 4,442,204 to Liotta, and U.S. Pat. No. 5,208,535 to Buechler, et al.

The antibodies can be labeled. In some embodiments, the detection antibody is labeled by covalently linking to an enzyme, label with a fluorescent compound or metal, label with a chemiluminescent compound. For example, the detection antibody can be labeled with catalase and the conversion uses a colorimetric substrate composition comprises potassium iodide, hydrogen peroxide and sodium thiosulphate; the enzyme can be alcohol dehydrogenase and the conversion uses a colorimetric substrate composition comprises an alcohol, a pH indicator and a pH buffer, wherein the pH indicator is neutral red and the pH buffer is glycine-sodium hydroxide; the enzyme can also be hypoxanthine oxidase and the conversion uses a colorimetric substrate composition comprises xanthine, a tetrazolium salt and 4,5-dihydroxy-1,3-benzene disulphonic acid. In one embodiment, the detection antibody is labeled by covalently linking to an enzyme, label with a fluorescent compound or metal, or label with a chemiluminescent compound.

Direct and indirect labels can be used in immunoassays. A direct label can be defined as an entity, which in its natural state, is visible either to the naked eye or with the aid of an optical filter and/or applied stimulation, e.g., ultraviolet light, to promote fluorescence. Examples of colored labels which can be used include metallic sol particles, gold sol particles, dye sol particles, dyed latex particles or dyes encapsulated in liposomes. Other direct labels include radionuclides and fluorescent or luminescent moieties. Indirect labels such as enzymes can also be used according to the present disclosure. Various enzymes are known for use as labels such as, for example, alkaline phosphatase, horseradish peroxidase, lysozyme, glucose-6-phosphate dehydrogenase, lactate dehydrogenase and urease. For a detailed discussion of enzymes in immunoassays see Engvall, Enzyme Immunoassay ELISA and EMIT, Methods of Enzymology, 70, 419-439 (1980).

The antibody can be attached to a surface. Examples of useful surfaces on which the antibody can be attached for the purposes of detecting the desired antigen include nitrocellulose, PVDF, polystyrene, and nylon. The surface or support may also be a porous support (see, e.g., U.S. Patent No. 7,939,342). The assays can be carried out in various assay device formats including those described in U.S. Pat. Nos. 4,906,439; 5,051,237 and 5,147,609 to PB Diagnostic Systems, Inc.

### Reference Values

In various embodiments of the assays and methods described herein, the reference value is based on the expression level of, for example, total IgG, subtypes of IgG or antigen specific IgG levels. In one embodiment, the expression level is the level in a sample obtained from a subject that has immune-mediated disease. In another embodiment, the expression level is the level in a sample from a subject that does not have immune-mediated disease. In some embodiments, the reference value is the mean or median expression level of total IgG or subtypes of IgG in a population of subjects that do not have immune-mediated disease.

In other embodiments, the reference value is the mean or median expression level of total IgG, subtypes of IgG or antigen specific IgG levels in a population of subjects that have immune-mediated diseases but are not selected for anti-FcRn therapy (for example IBD subjects that do not express the His131 allele of FcγR2a). In other embodiments, the reference value is the mean or median expression level of total IgG, subtypes of IgG or antigen specific IgG levels in a population of subjects that have IBD and respond to anti-FcRn therapy. In some embodiments the reference value that comprises the population of subjects that have immune-mediated disease and respond to anti-FcRn therapy show undetectable expression of total IgG (or subtypes of IgG) or show reduced expression of total IgG (or subtypes of IgG and antigen specific IgG). In additional embodiments, the reference value is the expression level of IgG in a sample obtained from the subject from a different (for example, an earlier) time point, such as during diagnosis, before treatment, after treatment or a combination thereof.

In various embodiments, the expression level of IgG in the subject diagnosed with immune-mediated disease wherein the subject expresses the His131 allele of FcγR2a compared to the reference value is increased by at least or about 10%, 20%, 30%, 40%, 50%, 60%, 70%, 80%, 90% or 100%. In various embodiments, the expression level of IgG in the subject diagnosed with immune-mediated disease compared to the reference value is increased by at least or about 1-fold, 2-fold, 3-fold, 4-fold, 5-fold, 10-fold, 15-fold, 20-fold, 25-fold, 30-fold, 35-fold, 40-fold, 45-fold, 50-fold, 55-fold, 60-fold, 65-fold, 70-fold, 75-fold, 80-fold, 85-fold, 90-fold, 95-fold, 100-fold, or more.

In various embodiments, a subject is deemed responsive to anti-FcRn therapy if the IgG expression level in a sample obtained from a subject undergoing anti-FcRn therapy is reduced compared to the reference value by at least or about 10%, 20%, 30%, 40%, 50%, 60%, 70%, 80%, 90% or 100%. In various embodiments, the expression level of IgG in the subject diagnosed with immune-mediated disease compared to the reference value is increased by at least or about 1-fold, 2-fold, 3-fold, 4-fold, 5-fold, 10-fold, 15-fold, 20-fold, 25-fold, 30-fold, 35-fold, 40-fold, 45-fold, 50-fold, 55-fold, 60-fold, 65-fold, 70-fold, 75-fold, 80-fold, 85-fold, 90-fold, 95-fold, 100-fold , or more.

### Therapies

As described herein, in some embodiments, patients with immune-mediated diseases that express the His131 allele of FcγR2a may be administered effective amounts of compositions comprising anti-FcRn agents so as to treat the immune-mediated disease, inhibit immune-mediated disease or reduce the severity of immune-mediated disease in the patients. In various embodiments, the anti-FcRn agents may comprise an agent that blocks FcRn or an agent that reduces or inhibits expression of FcRn, or a combination thereof. In some embodiments, agents that block FcRn may be any one or more of a peptide, protein, small molecule, nucleic acid, aptamer, oligonucleotide, antibody or a combination thereof. The nucleic acid may be a siRNA specific to FcRn.

In some aspects, the anti-FcRn agent in the composition includes but is not limited to any one or more of antibodies ("antibodies" includes antigen-binding portions of antibodies such as epitope- or antigen-binding peptides, paratopes, functional CDRs; recombinant antibodies; chimeric antibodies; tribodies; midibodies; or antigen-binding derivatives, analogs, variants, portions, or antigen-binding fragments thereof), protein-binding agents, small molecules, recombinant protein, peptides, aptamers, avimers and protein-binding derivatives, portions or fragments thereof, or combinations thereof.

Antisense oligonucleotides represent another class of agents that are useful in the compositions and methods described herein, particularly as FcRn antagonists. This class of agents and methods for preparing and using them are all well-known in the art, as are ribozyme and miRNA molecules. See, e.g., PCT US2007/024067 for a thorough discussion. Alternatively, the anti-FcRn agent in some embodiments of the compositions and methods described herein, include recombinant Fc or conjugates, or protein or antibody, small interfering RNA specific for or targeted to FcRn mRNA, and antisense RNA that hybridizes with the mRNA of FcRn, for example.

Other agents for use in the compositions and methods described herein that block FcRn include, for example, antibodies against FcRn, specifically reactive or specifically binding to FcRn. In some embodiments, the antibody is a blocking antibody and may be any of a monoclonal antibody or a fragment thereof, a polyclonal antibody or a fragment thereof, a chimeric antibody, humanized antibody or a single chain antibody.

In some embodiments, the agent is a bispecific agent comprises binding sites for Fcγ receptors and FcRn. In some embodiments, an agent targets FcRn and a second, different agent targets the Fcγ receptors and the two agents may be administered simultaneously or sequentially.

In some embodiments, the agent is a bispecific agent and comprises binding sites for IgG and Fcγ receptors. In some embodiments, the agent is a bispecific agent and comprises binding sites for IgG and FcRn. In various embodiments, distinct agents may target IgG, Fcγ receptors and FcRn and each of these agents may be administered sequentially or simultaneously.

In some embodiments, the agent is a recombinant Fc portion of IgG or a biologically active portion thereof or a proteo-mimetic thereof that binds FcRn. The Fc portion or a biologically active portion thereof may be mammalian. The Fc portion or a biologically active portion thereof may be human.

As used herein, a "blocking" antibody or an antibody "antagonist" is one which inhibits or reduces the biological activity of the antigen(s) it binds. For example, a FcRn blocking or antagonist antibody binds FcRn and inhibits or reduces the function and/or interactions of FcRn. In an embodiment, the monoclonal antibody that specifically binds FcRn is DVN24 (Christianson et al., Landes Bioscience 2012 Vol 4:2 pp208-216). In an embodiment, DVN24 is humanized and affinity matured.

Simple binding assays can be used to screen for or detect agents that bind to FcRn. Further, agents that block or inhibit FcRn for use in the compositions and methods described herein, including recombinant FcRn peptido-mimetics, can be identified by, for example, transfecting cells with expression vectors expressing FcRn or portions thereof; contacting the cells with an agent; lysing the cells; and characterizing the FcRn interactions in comparison with cells not contacted with agent. Cells can be characterized using, for example, co-immunoprecipitation.

Another variation of assays to determine binding of a FcRn blocking protein is through the use of affinity biosensor methods. Such methods may be based on the piezoelectric effect, electrochemistry, or optical methods, such as ellipsometry, optical wave guidance, and surface plasmon resonance (SPR). For example, efficacy of an siRNA on the expression of FcRn can be monitored using methods known in the art such as quantitative RT-PCR with specific oligonucleotide primers for each gene respectively, or ELISA for FcRn from a sample of blood. Alternatively, the population of blood cells can be determined by flow cytometric analysis using the markers characteristic of particular populations and subpopulations known in the art or disclosed herein.

The term "effective amount" as used herein refers to the amount of an agent for blocking or inhibiting FcRn needed to alleviate at least one or more symptom of the disease or disorder, and relates to a sufficient amount of pharmacological composition to provide the desired effect, for example, treating IBD. The term "therapeutically effective amount" therefore refers to an amount of an agent for blocking FcRn using the methods as disclosed herein, that is sufficient to effect a particular effect when administered to a typical subject. An effective amount as used herein would also include an amount sufficient to delay the development of a symptom of the disease, alter the course of a symptom of disease (for example but not limited to, slow the progression of a symptom of the disease), or reverse a symptom of disease. Thus, it is not possible to specify the exact "effective amount". However, for any given case, an appropriate "effective amount" can be determined by one of ordinary skill in the art using only routine experimentation.

Effective amounts, toxicity, and therapeutic efficacy can be determined by standard pharmaceutical procedures in cell cultures or experimental animals, *e.g.*, for determining the LD50 (the dose lethal to 50% of the population) and the ED50 (the dose therapeutically effective in 50% of the population). The dosage can vary depending upon the dosage form employed and the route of administration utilized. The dose ratio between toxic and therapeutic effects is the therapeutic index and can be expressed as the ratio LD50/ED50. Compositions and methods that exhibit large therapeutic indices are preferred. A therapeutically effective dose can be estimated initially from cell culture assays. Also, a dose can be formulated in animal models to achieve a circulating plasma concentration range that includes the IC50 (*i.e.*, the concentration of the agent for blocking FcRn, which achieves a half-maximal inhibition of symptoms) as determined in cell culture, or in an appropriate animal model. Levels in plasma can be measured, for example, by high performance liquid chromatography. The effects of any particular dosage can be monitored by a suitable bioassay. The dosage can be determined by a physician and adjusted, as necessary, to suit observed effects of the treatment.

The agents useful according to the compositions and methods described herein, including antibodies and other polypeptides, are isolated agents, meaning that the agents are substantially pure and are essentially free of other substances with which they may be found in nature or *in vivo* systems to an extent practical and appropriate for their intended use. In particular, the agents are sufficiently pure and are sufficiently free from other biological constituents of their host cells so as to be useful in, for example, producing pharmaceutical preparations. Because an isolated agent may be admixed with a pharmaceutically acceptable carrier in a pharmaceutical preparation, the agents may comprise only a small percentage by weight of the preparation.

The agents described herein for anti-FcRn therapy can be administered to a subject in need thereof by any appropriate route which results in an effective treatment in the subject. As used herein, the terms "administering," and "introducing" are used interchangeably and refer to the placement of an agent into a subject by a method or route which results in at least partial localization of such agents at a desired site, such as a site of inflammation, such that a desired effect(s) is produced.

In some embodiments, the agents described herein for use in anti-FcRn therapy are administered to a subject by any mode of administration that delivers the agent systemically or to a desired surface or target, and can include, but is not limited to, injection, infusion, instillation, and inhalation administration. To the extent that polypeptide agents can be protected from inactivation in the gut, oral administration forms are also contemplated. "Injection" includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion. In some embodiments, the agents for use in anti-FcRn therapy for use in the methods described herein are administered by intravenous infusion or injection.

The phrases "parenteral administration" and "administered parenterally" as used herein, refer to modes of administration other than enteral and topical administration, usually by injection. The phrases "systemic administration," "administered systemically", "peripheral administration" and "administered peripherally" as used herein refer to the administration of an anti-FcRn agent other than directly into a target site, tissue, or organ, such that it enters the subject's circulatory system and, thus, is subject to metabolism and other like processes.

For the clinical use of the methods described herein, administration of an anti-FcRn can include formulation into pharmaceutical compositions or pharmaceutical formulations for parenteral administration, *e.g.*, intravenous; mucosal, *e.g.*, intranasal; ocular, or other mode of administration. In some embodiments, an anti-FcRn agent can be administered along with any pharmaceutically acceptable carrier compound, material, or composition which results in an effective treatment in the subject. Thus, a pharmaceutical formulation for use in the methods described herein can contain an anti-FcRn agent in combination with one or more pharmaceutically acceptable ingredients.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically acceptable carrier" as used herein means a pharmaceutically acceptable material, composition or vehicle, such as a liquid or solid filler, diluent, excipient, solvent, media, encapsulating material, manufacturing aid (*e.g.*, lubricant, talc magnesium, calcium or zinc stearate, or steric acid), or solvent encapsulating material, involved in maintaining the stability, solubility, or activity of, an agent for modulating interactions between FcRn and IgG or a fragment thereof or a variant thereof. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) excipients, such as cocoa butter and suppository waxes; (8) oils, such as peanut oil, cottonseed oil, safflower oil, sesame oil, olive oil, corn oil and soybean oil; (9) glycols, such as propylene glycol; (10) polyols, such as glycerin, sorbitol, mannitol and polyethylene glycol (PEG); (11) esters, such as ethyl oleate and ethyl laurate; (12) agar; (13) buffering agents, such as magnesium hydroxide and aluminum hydroxide; (14) alginic acid; (15) pyrogen-free water; (16) isotonic saline; (17) Ringer's solution; (18) pH buffered solutions; (19) polyesters, polycarbonates and/or polyanhydrides; (20) bulking agents, such as polypeptides and amino acids (21) serum components, such as serum albumin, HDL and LDL; (22) C2-C12 alchols, such as ethanol; and (23) other non-toxic compatible substances employed in pharmaceutical formulations. Release agents, coating agents, preservatives, and antioxidants can also be present in the formulation. The terms such as "excipient", "carrier", "pharmaceutically acceptable carrier" or the like are used interchangeably herein.

The anti-FcRn agents described herein can be specially formulated for administration of the compound to a subject in solid, liquid or gel form, including those adapted for the following: (1) parenteral administration, for example, by subcutaneous, intramuscular, intravenous or epidural injection as, for example, a sterile solution or suspension, or sustained-release formulation; (2) topical application, for example, as a cream, ointment, or a controlled-release patch or spray applied to the skin; (3) intravaginally or intrarectally, for example, as a pessary, cream or foam; (4) ocularly; (5) transdermally; (6) transmucosally; or (7) nasally. Additionally, the anti-FcRn agent can be implanted into a patient or injected using a drug delivery system. See, for example, Urquhart, et al., Ann. Rev. Pharmacol. Toxicol. 24: 199-236 (1984); Lewis, ed. "Controlled Release of Pesticides and Pharmaceuticals" (Plenum Press, New York, 1981); U.S. Pat. No. 3,773,919; and U.S. Pat. No. 35 3,270,960.

Further embodiments of the formulations and modes of administration of an anti-FcRn agent that can be used in the methods described herein are illustrated below.

Parenteral Dosage Forms. Parenteral dosage forms of an anti-FcRn agent can also be administered to a subject by various routes, including, but not limited to, subcutaneous, intravenous (including bolus injection), intramuscular, and intraarterial. Since administration of parenteral dosage forms typically bypasses the patient's natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, controlled-release parenteral dosage forms, and emulsions.

Suitable vehicles that can be used to provide parenteral dosage forms of the disclosure are well known to those skilled in the art. Examples include, without limitation: sterile water; water for injection USP; saline solution; glucose solution; aqueous vehicles such as but not limited to, sodium chloride injection, Ringer's injection, dextrose Injection, dextrose and sodium chloride injection, and lactated Ringer's injection; water-miscible vehicles such as, but not limited to, ethyl alcohol, polyethylene glycol, and propylene glycol; and non-aqueous vehicles such as, but not limited to, corn oil, cottonseed oil, peanut oil, sesame oil, ethyl oleate, isopropyl myristate, and benzyl benzoate.

Aerosol formulations. An anti-FcRn agent can be packaged in a pressurized aerosol container together with suitable propellants, for example, hydrocarbon propellants like propane, butane, or isobutane with conventional adjuvants. An anti-FcRn agent can also be administered in a non-pressurized form such as in a nebulizer or atomizer. An anti-FcRn can also be administered directly to the airways in the form of a dry powder, for example, by use of an inhaler.

Suitable powder compositions include, by way of illustration, powdered preparations of an anti-FcRn agent thoroughly intermixed with lactose, or other inert powders acceptable for intrabronchial administration. The powder compositions can be administered via an aerosol dispenser or encased in a breakable capsule which can be inserted by the subject into a device that punctures the capsule and blows the powder out in a steady stream suitable for inhalation. The compositions can include propellants, surfactants, and co-solvents and can be filled into conventional aerosol containers that are closed by a suitable metering valve.

Aerosols for the delivery to the respiratory tract are known in the art. See for example, Adjei, A. and Garren, J. Pharm. Res., 1: 565-569 (1990); Zanen, P. and Lamm, J.-W. J. Int. J. Pharm., 114: 111-115 (1995); Gonda, I. "Aerosols for delivery of therapeutic an diagnostic agents to the respiratory tract," in Critical Reviews in Therapeutic Drug Carrier Systems, 6:273-313 (1990); Anderson et al., Am. Rev. Respir. Dis., 140: 1317-1324 (1989)) and have potential for the systemic delivery of peptides and proteins as well (Patton and Platz, Advanced Drug Delivery Reviews, 8:179-196 (1992)); Timsina et. al., Int. J. Pharm., 101: 1-13 (1995); and Tansey, I. P., Spray Technol. Market, 4:26-29 (1994); French, D. L., Edwards, D. A. and Niven, R. W., Aerosol Sci., 27: 769-783 (1996); Visser, J., Powder Technology 58: 1-10 (1989)); Rudt, S. and R. H. Muller, J. Controlled Release, 22: 263-272 (1992); Tabata, Y, and Y. Ikada, Biomed. Mater. Res., 22: 837-858 (1988); Wall, D. A., Drug Delivery, 2: 10 1-20 1995); Patton, J. and Platz, R., Adv. Drug Del. Rev., 8: 179-196 (1992); Bryon, P., Adv. Drug. Del. Rev., 5: 107-132 (1990); Patton, J. S., et al., Controlled Release, 28: 15 79-85 (1994); Damms, B. and Bains, W., Nature Biotechnology (1996); Niven, R. W., et al., Pharm. Res., 12(9); 1343-1349 (1995); and Kobayashi, S., et al., Pharm. Res., 13(1): 80-83 (1996).

The formulations of the anti-FcRn agents described herein further encompass anhydrous pharmaceutical compositions and dosage forms comprising the disclosed compounds as active ingredients, since water can facilitate the degradation of some compounds. For example, the addition of water (*e.g.*, 5%) is widely accepted in the pharmaceutical arts as a means of simulating long-term storage in order to determine characteristics such as shelf life or the stability of formulations overtime. See, *e.g.*, Jens T. Carstensen, Drug Stability: Principles & Practice, 379-80 (2nd ed., Marcel Dekker, NY, N.Y.: 1995). Anhydrous pharmaceutical compositions and dosage forms of the disclosure can be prepared using anhydrous or low moisture containing ingredients and low moisture or low humidity conditions. Pharmaceutical compositions and dosage forms that comprise lactose and at least one active ingredient that comprise a primary or secondary amine are preferably anhydrous if substantial contact with moisture and/or humidity during manufacturing, packaging, and/or storage is expected. Anhydrous compositions are preferably packaged using materials known to prevent exposure to water such that they can be included in suitable formulary kits. Examples of suitable packaging include, but are not limited to, hermetically sealed foils, plastics, unit dose containers (e.g., vials) with or without desiccants, blister packs, and strip packs.

Controlled and Delayed Release Dosage Forms. In some embodiments of the methods described herein, an anti-FcRn agent can be administered to a subject by controlled- or delayed-release means. Ideally, the use of an optimally designed controlled-release preparation in medical treatment is characterized by a minimum of drug substance being employed to cure or control the condition in a minimum amount of time. Advantages of controlled-release formulations include: 1) extended activity of the drug; 2) reduced dosage frequency; 3) increased patient compliance; 4) usage of less total drug; 5) reduction in local or systemic side effects; 6) minimization of drug accumulation; 7) reduction in blood level fluctuations; 8) improvement in efficacy of treatment; 9) reduction of potentiation or loss of drug activity; and 10) improvement in speed of control of diseases or conditions. (Kim, Cherng-ju, Controlled Release Dosage Form Design, 2 (Technomic Publishing, Lancaster, Pa.: 2000)). Controlled-release formulations can be used to control a compound's onset of action, duration of action, plasma levels within the therapeutic window, and peak blood levels. In particular, controlled- or extended-release dosage forms or formulations can be used to ensure that the maximum effectiveness of a compound of formula (I) is achieved while minimizing potential adverse effects and safety concerns, which can occur both from under-dosing a drug (*i.e.*, going below the minimum therapeutic levels) as well as exceeding the toxicity level for the drug.

A variety of known controlled- or extended-release dosage forms, formulations, and devices can be adapted for use with the anti-FcRn agents described herein. Examples include, but are not limited to, those described in U.S. Pat. Nos.: 3,845,770; 3,916,899; 3,536,809; 3,598,123; 4,008,719; 5674,533; 5,059,595; 5,591,767; 5,120,548; 5,073,543; 5,639,476; 5,354,556; 5,733,566; and 6,365,185 B1. These dosage forms can be used to provide slow or controlled-release of one or more active ingredients using, for example, hydroxypropylmethyl cellulose, other polymer matrices, gels, permeable membranes, osmotic systems (such as OROS® (Alza Corporation, Mountain View, Calif. USA)), multilayer coatings, microparticles, liposomes, or microspheres or a combination thereof to provide the desired release profile in varying proportions. Additionally, ion exchange materials can be used to prepare immobilized, adsorbed salt forms of the disclosed compounds and thus effect controlled delivery of the drug. Examples of specific anion exchangers include, but are not limited to, Duolite® A568 and Duolite® AP143 (Rohm&Haas, Spring House, Pa. USA).

In some embodiments, an anti-FcRn agent for use in the methods described herein is administered to a subject by sustained release or in pulses. Pulse therapy is not a form of discontinuous administration of the same amount of a composition over time, but comprises administration of the same dose of the composition at a reduced frequency or administration of reduced doses. Sustained release or pulse administrations are particularly preferred when the disorder occurs continuously in the subject, for example where the subject has continuous or chronic symptoms of a viral infection. Each pulse dose can be reduced and the total amount of the anti-FcRn agent administered over the course of treatment to the patient is minimized.

The interval between pulses, when necessary, can be determined by one of ordinary skill in the art. Often, the interval between pulses can be calculated by administering another dose of the composition when the composition or the active component of the composition is no longer detectable in the subject prior to delivery of the next pulse. Intervals can also be calculated from the *in vivo* half-life of the composition. Intervals can be calculated as greater than the *in vivo* half-life, or 2, 3, 4, 5 and even 10 times greater the composition half-life. Various methods and apparatus for pulsing compositions by infusion or other forms of delivery to the patient are disclosed in U.S. Pat. Nos. 4,747,825; 4,723,958; 4,948,592; 4,965,251 and 5,403,590.

### EXAMPLES

The following examples are provided to better illustrate the claimed invention and are not to be interpreted as limiting the scope of the invention. To the extent that specific materials are mentioned, it is merely for purposes of illustration and is not intended to limit the invention. One skilled in the art may develop equivalent means or reactants without the exercise of inventive capacity and without departing from the scope of the invention, which is as defined by the appended claims.

As described herein, the H131 variant of FcγR2a is a genetic risk allele for several immune mediated diseases in both Caucasians and non-Caucasians such that individuals with this allele exhibit a higher risk for a specific immune-mediated disease. In addition to exhibiting a higher affinity for IgG2, the His variant of FcγR2a exhibits much more strong signaling when bound to IgG2 and IgG1 immune complexes and this is associated with increased production of inflammatory cytokines and enhancement of both MHC class I and MHC class II presentation of immune complex bound antigens. Among the cytokines that are increased by the FcγR2a His131 (also known as His167 according to NM_001136219.1) allele is IL-12.

The increased signaling and antigen presentation and thus activation of T cells which is associated with excessive immune complex binding and signaling by the His allele is abrogated by blockade or elimination of FcRn expression by antigen presenting cells. This predicts and as described herein, any individual with the FcγR2a-His131 (also known as His167 according to NM_001136219.1) allele will exhibit much greater IgG driven inflammation and that blockade of inflammation with FcRn-targeted therapies will eliminate this inflammation.

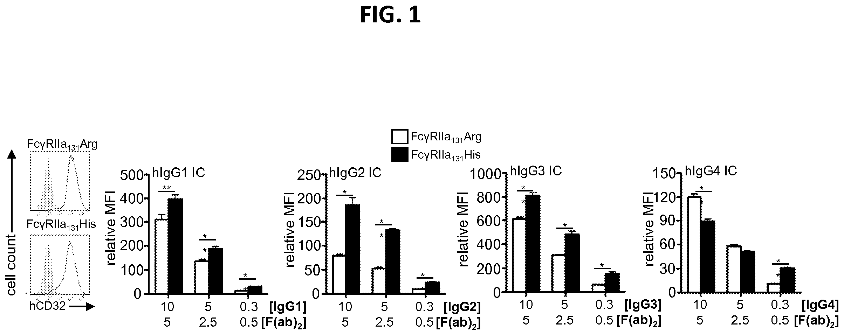

The binding of FcγR2a131His to immune complexes was analyzed. Madin-Darby canine kidney (MDCK) cells were transfected with vectors expressing FcγR2a131Arg allele, FcγR2a131His allele or the control vector. Binding of human immune complexes consisting of F(ab)₂ fragments complexed with IgG of different subclasses was assessed at the indicated concentration by Flow. As shown in Figure 1, FcγR2a131His allele binds human immune complexes more efficiently than FcγR2a131Arg.

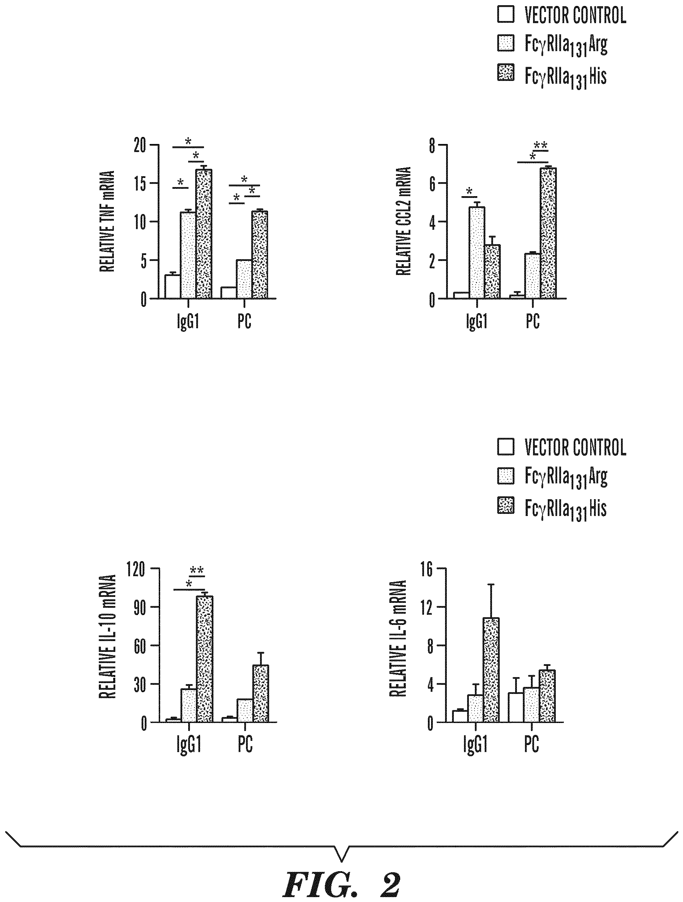

The effect of FcγR2a131His on cytokine expression was assessed. Mouse leukaemic monocyte macrophage (RAW 264.7) cells were stably transfected with vectors expressing FcγR2a131Arg allele, FcγR2a131His allele or the control vector. The transfected cells were stimulated with IgG1-NIP conjugated OVA immune complexes (IgG1) or polymeric NIP-OVA immune complexes (PC). Immune complexes were generated with a chimeric antibody that contained a Fab region that is specific for NIP (SM Claypool, Mol Biol Cell 2004 15:1746-59). Cytokine production was assessed by qPCR. Binding of human immune complexes consisting of F(ab)₂ fragments complexed with IgG of different subclasses was assessed at indicated concentration by Flow. As shown in Figure 2, FcγR2a131His mediates increased cytokine expression upon stimulation with immune complexes compared to FcγR2a131Arg.

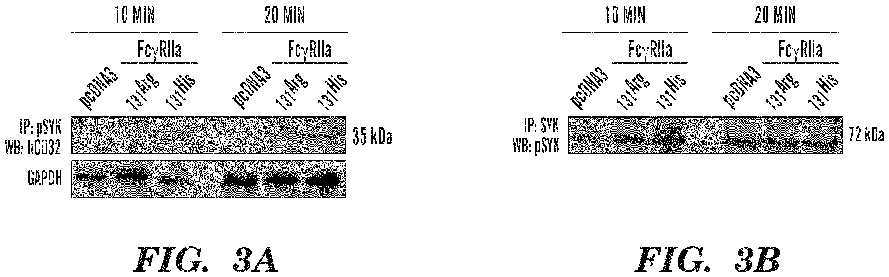

The effect of FcγR2a131His on downstream signaling was assayed. RAW 264.7 macrophages were stably transfected with FcγR2a131Arg, FcγR2a131His, or vector control and stimulated with IgG1-NIP OVA Immune complexes for the indicated time points. The early adapter molecule phosphorylated SYK (pSYK) was precipitated first and bound hCD32 was detected by western blotting afterwards (Figure 3a). Total cellular SYK was precipitated first and phosphorylated SYK (pSYK) detected by western blotting afterwards (Figure 3b). As shown in Figure 3, FcγR2a131His exhibits increased downstream signaling upon stimulation with immune complexes compared to FcγR2a131Arg.

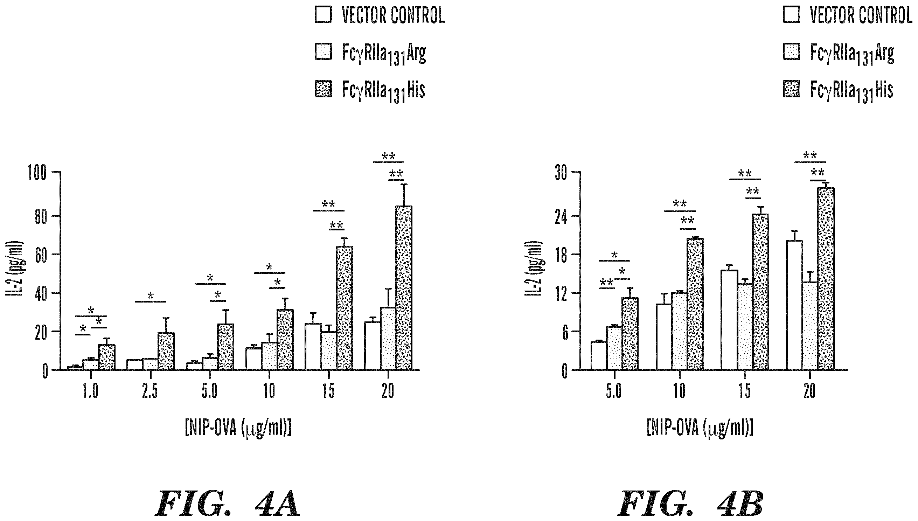

The His variant of FcγR2a exhibits much more strong signaling when bound to IgG2 and IgG1 immune complexes and this is associated with increased production of inflammatory cytokines and enhancement of both MHC class I and MHC class II presentation of immune complex bound antigens. For MHC II antigen presentation in Figure 4A, RAW 264.7 macrophages were stably transfected with FcγR2a131Arg, FcγR2a131His, or vector control and stimulated with IgG1-NIP OVA immune complexes at the indicated concentrations. After 3 hours, OVA specific CD4+ T cells were added and incubated for 24 hours and IL-2 release from CD4+ T cells was measured by ELISA. For MHC I cross-presentation in Figure 4B, H2-k^{b} expressing HEK293T cells macrophages were stably transfected with FcγR2a131Arg, FcγR2a131His, or vector control and stimulated with IgG1-NIP OVA immune complexes at the indicated concentrations. After 3 hours, OVA specific CD8+ T cells were added and incubated for 24 hours and IL-2 release from CD4+ T cells was measured by ELISA. As shown in Figure 4, FcγR2a131His induces MHC II presentation and MHC I cross-presentation of immune complexed antigen more efficiently than FcγR2a131Arg.

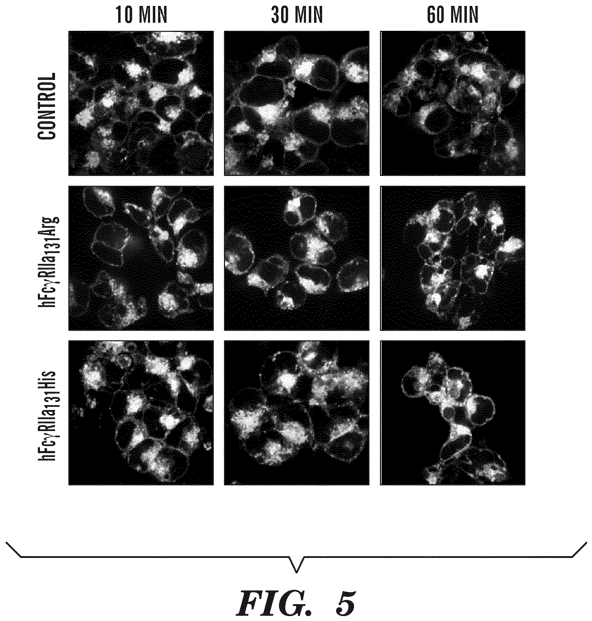

HEK293 cells expressing GFP tagged human FcRn were stably transfected with vectors expressing FcγR2a131Arg allele, FcγR2a131His allele or the control vector. The transfected cells were stimulated with human IgG2 containing immune complexes. As shown in Figure 5, FcγR2a131His internalizes immune complexes more efficiently than FcγR2a131Arg and transfers them into a FcRn containing intracellular compartment.

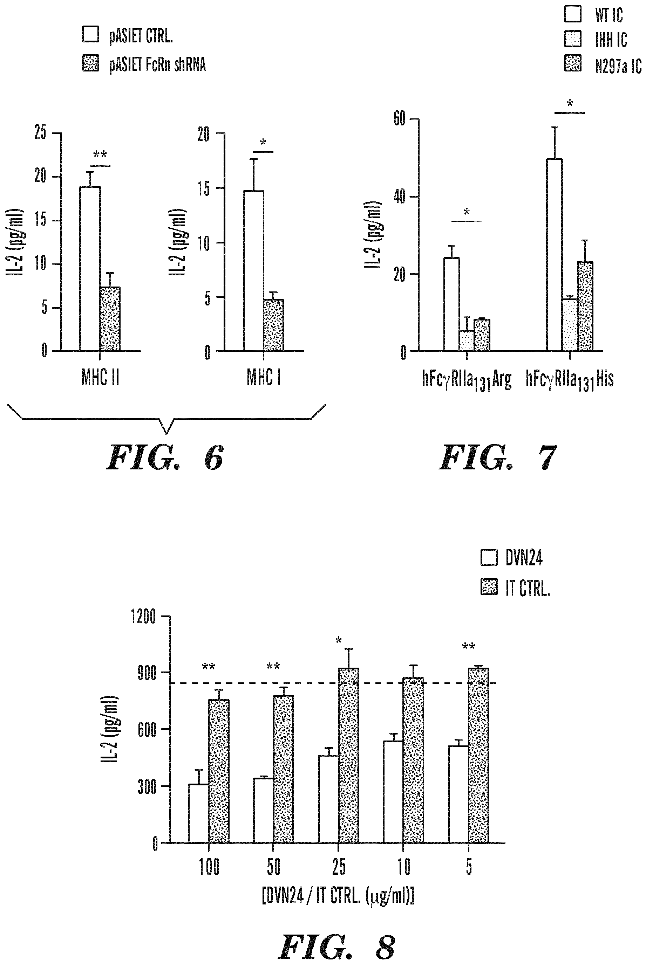

The role of FcRn-mediated presentation of MHC II and cross-presentation of MHC I was assessed as a function of IL-2 release. JAWS II dendritic cells were stably lentivirally transduced with an shRNA silencing FcRn expressing or control virus. Cells were stimulated with IgG1 containing NIP-OVA immune complexes for 3 hours and antigen specific CD4+ T cells (left panel) or antigen specific CD8+ T cells (right panel were added). IL-2 release was measured by ELISA. As shown in Figure 6, FcRn is a critical mediator for MHC II antigen presentation and MHC I cross-presentation if immune complexed antigen is provided.

RAW macrophages stably expressing FcγR2a131ARG or FcγR2a131His were stimulated with wild type IgG1NIP-OVA immune complexes (WT), non-FcRn binding IgG1NIP-OVA immune complexes (IHH) or non-FcγR binding immune complexes (N297a) for 3 hours. Afterwards, antigen-specific CD4+ T cells were added and IL-2 release from CD4+ T cells was measured by ELISA. As shown in Figure 7, enhanced FcγR2a initiated antigen presentation is abrogated by loss of FnRn or FcγR binding.

Bone marrow derived dendritic cells were pre-incubated DVN24, an antibody blocking the interaction between IgG and FcRn. Bone marrow derived dendritic cells were pre-incubated DVN24, an antibody blocking the interaction between IgG and FcRn. As shown in Figure 8, over a broad concentration range, DVN24 significantly reduces MHC II presentation of immune complex derived antigen compared to isotype control as indicated by IL-2 release from antigen specific CD4+ T cells.

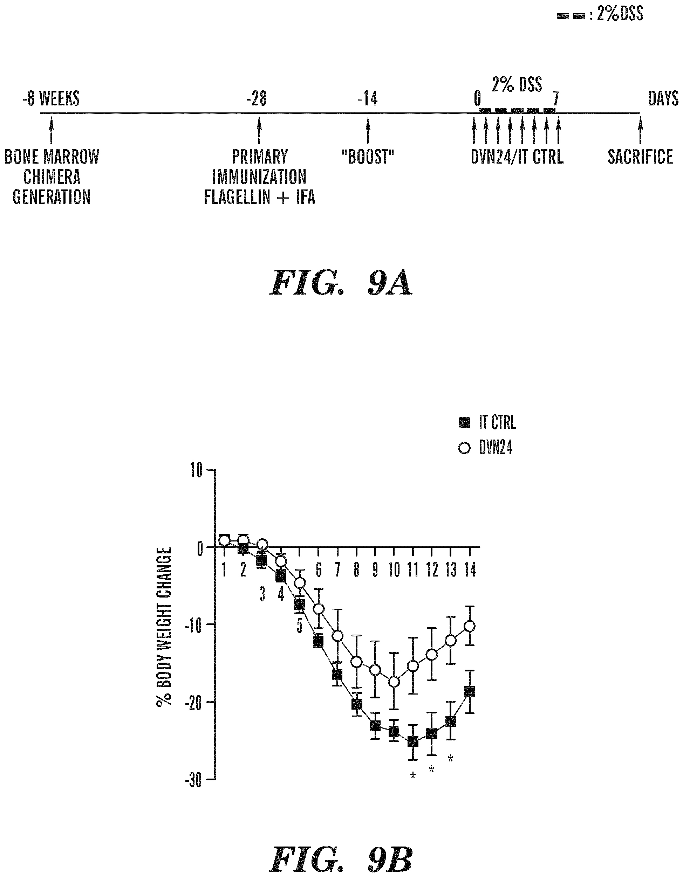

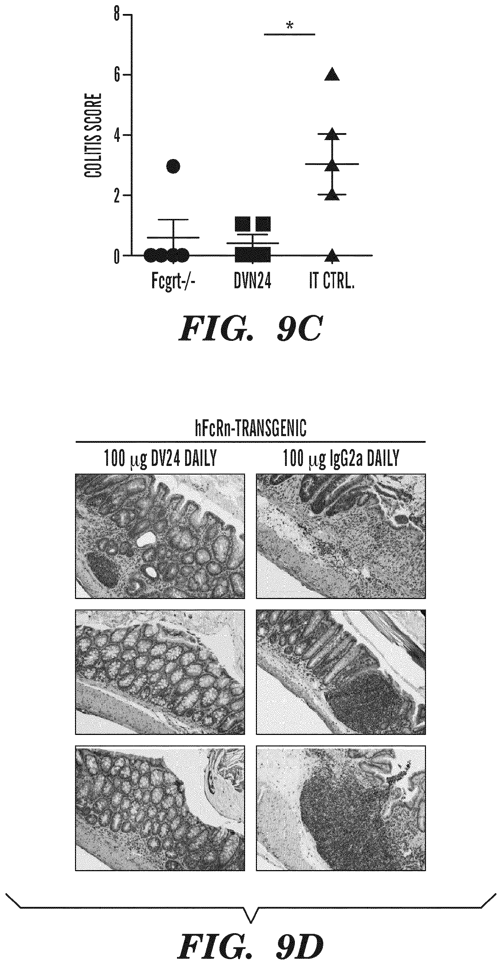

Bone marrow chimera mice were generated (Figure 9a) and immunized with bacterial Flagellin to induce high titers of anti-bacterial IgG. During subsequent dextran sodium sulfate colitis, mice that received a daily dose of 100 ug DVN24 ip were relatively protected from the development of colitis compared to isotype control treated mice, as assessed by body weight change (Figure 9b) and histologic colitis scoring (Figures 9c and d). As shown in Figure 9, pharmacologic blockade of hFcRn with DVN24 in hematopoietic cells protected from the development of IgG mediated colitis.

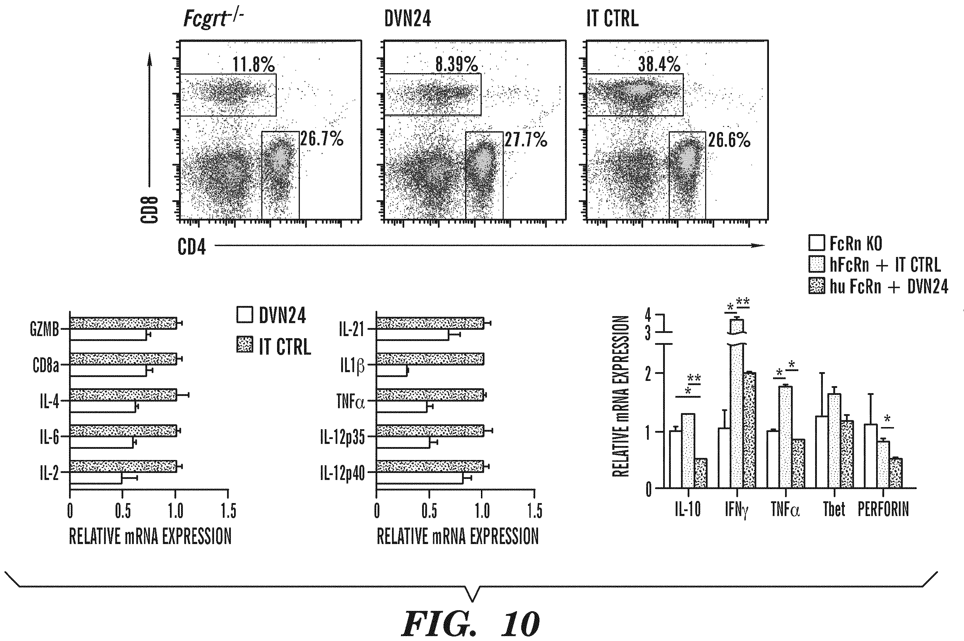

The effect of FcRn blockade on CD8⁺ T cells and inflammatory cytokines was assessed. Mice expressing human FcRn in the bone marrow compartment, or bone marrow chimeric mice without FcRn expression in the bone marrow, were immunized with flagellin to induce high titers of anti-bacterial IgG. During subsequent dextran sodium sulfate colitis, human FcRn chimeric mice that received a daily dose of 100 ug DVN24 ip exhibited lower CD8+ T cell numbers in the lamina propria compared to human FcRn chimeric mice that were treated with an isotype control or compared to mice without FcRn expression in the hematopoietic compartment (Figure 10, upper panels). Further, human FcRn chimeric mice treated with DVN24 ip during colitis had significantly lower expression of pro-inflammatory cytokines in the total colon (Figure 10 lower left panels) and from the lamina propria lymphocyte fraction (Figure 10 lower right panel) compared to isotype control treated mice. These data show that pharmacological blockade of FcRn with the DVN24 monoclonal antibody decreases the number of CD8+ T cells and expression of pro-inflammatory cytokines in the gut.

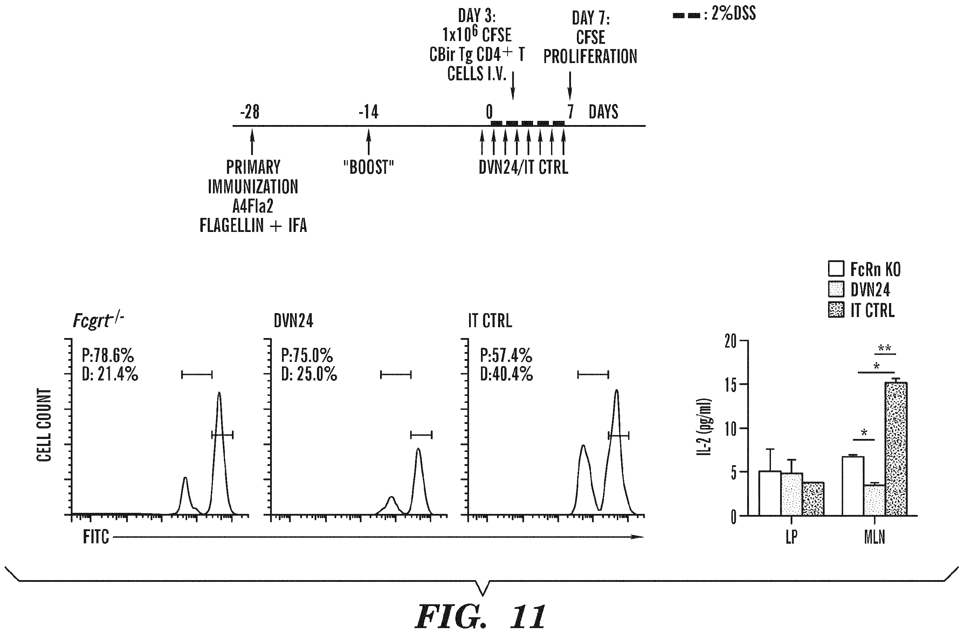

The effect of FnRn blockade on CD4⁺ T cells was assessed. Proliferation of CFSE labeled CBir Flagellin specific CD4⁺ T cells isolated from the lamina propria of *hFCGRT*/*hβ2M*/*mFcgrt*^{*-*/*-*} BM chimeras treated with DVN24 or an isotype control during DSS colitis and *mFcgrt*^{*-*/*-*} BM chimeras treated with PBS during DSS colitis. IL-2 production from CBir Flagellin specific CD4⁺ T cells in an *ex vivo* antigen presentation assay with CD11c⁺DC's isolated from the lamina propria (LP) or the mesenteric lymph nodes (MLN) of *hFCGRT*/*hβ2M*/*mFcgrt*^{*-*/*-*} BM chimeras treated with DVN24 or an isotype control during DSS colitis and *mFcgrt^{-l-}* BM chimeras treated with PBS during DSS colitis as antigen presenting cells. As shown in Figure 11, pharmacological inhibition of FcRn with the DVN24 monoclonal antibody decreases commensal-specific CD4+ T cell activation in IgG-dependent colitis.

The functional binding of human FcRn to mouse IgG containing immune complexes was assessed. Human monocyte derived DC were incubated with IgG IC or FcRn-non-binding IHH-IgG for the indicated times. Cells were lysed and precipitated with anti-CD16/CD32 (anti-FcγRIII/FcgRII). Precipitated proteins were resolved by SDS-PAGE gel and transferred to a nitrocellulose membrane for Western blotting. The membrane was probed with anti-hFCGRT antibody. As shown in Figure 12, binding of FcRn by multimeric IgG immune complexes leads to prolonged interaction with FcγRs.

The inventors analyzed the co-association of human FcRn and human FcγR in antigen presenting cells. Bone marrow derived CD11c⁺ DCs isolated from WT, *Fcgrt*^{*-*/*-*} and *FCGRT*/*hB2M*/*mFcgrt*^{*-*/*-*} were used as antigen presenting cells. DCs were exposed to NIP-OVA complexed with polyclonal mouse IgG, washed and cocultured with OVA-specific CD4⁺ T cells. As shown in Figure 13, human FcRn mediates antigen presentation to CD4⁺ T cells as efficiently as WT FcRn in response to murine IgG immune complexes.

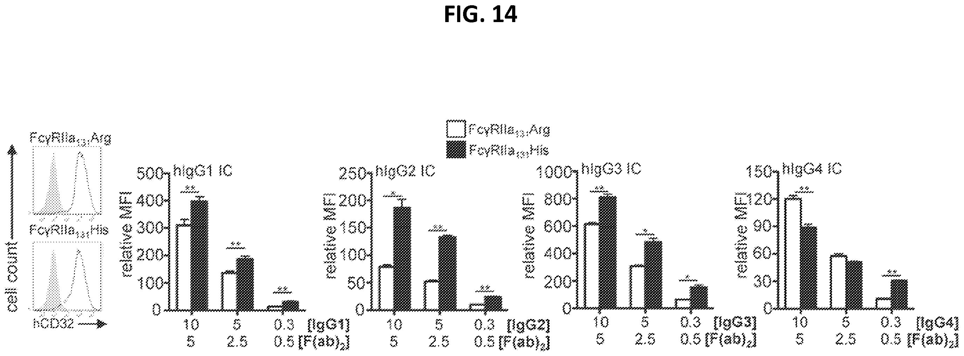

The binding of hFcγR2a₁₃₁His and hFcγR2a₁₃₁Arg to human IgG isotypes was assessed. Binding of fluorescently labeled human IgG immune complexes of various isotypes towards hFcγRIIa variants expressed on the cell surface of MDCK cells at neutral pH. As shown in Figure 14, hFcγR2a₁₃₁His exhibits stronger binding than hFcγR2a₁₃₁Arg across numerous IgG isotypes.

The various methods and techniques described above provide a number of ways to carry out the application. Of course, it is to be understood that not necessarily all objectives or advantages described can be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as taught or suggested herein. A variety of alternatives are mentioned herein. It is to be understood that some preferred embodiments specifically include one, another, or several features, while others specifically exclude one, another, or several features, while still others mitigate a particular feature by inclusion of one, another, or several advantageous features.

Furthermore, a person of ordinary skill in the art will recognize the applicability of various features from different embodiments. Similarly, the various elements, features and steps discussed above, as well as other known equivalents for each such element, feature or step, can be employed in various combinations by one of ordinary skill in this art to perform methods in accordance with the principles described herein. Among the various elements, features, and steps some will be specifically included and others specifically excluded in diverse embodiments.

Although the application has been disclosed in the context of certain embodiments and examples, it will be understood by those skilled in the art that the embodiments of the application extend beyond the specifically disclosed embodiments to other alternative embodiments and/or uses and modifications and equivalents thereof.

In some embodiments, the terms "a" and "an" and "the" and similar references used in the context of describing a particular embodiment of the application (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (for example, "such as") provided with respect to certain embodiments herein is intended merely to better illuminate the application and does not pose a limitation on the scope of the application otherwise claimed. No language in the specification should be construed as indicating any non-claimed element essential to the practice of the application.

Preferred embodiments of this application are described herein, including the best mode known to the inventors for carrying out the application. Variations on those preferred embodiments will become apparent to those of ordinary skill in the art upon reading the foregoing description. It is contemplated that skilled artisans can employ such variations as appropriate, and the application can be practiced otherwise than specifically described herein. Accordingly, many embodiments of this application include all modifications and equivalents of the subject matter recited in the claims appended hereto as permitted by applicable law. Moreover, any combination of the above-described elements in all possible variations thereof is encompassed by the application unless otherwise indicated herein or otherwise clearly contradicted by context.

It is to be understood that the embodiments of the application disclosed herein are illustrative of the principles of the embodiments of the application. Other modifications that can be employed can be within the scope of the application. Thus, by way of example, but not of limitation, alternative configurations of the embodiments of the application can be utilized in accordance with the teachings herein. Accordingly, embodiments of the present application are not limited to that precisely as shown and described.

## Claims

1. An assay comprising:
(i) assaying a biological sample obtained from a subject suspected of having an immune-mediated disease **characterised by** elevated IgG levels to determine the allele at position 131 of the FcγR2a protein;and
(ii) selecting the subject for a therapy if the subject expresses the His131 allele of FcγR2a, wherein the therapy comprises an antibody that blocks the neonatal Fc receptor (FcRn).

2. The assay of claim 1, wherein assaying the sample to determine the allele of FcγR2a comprises:
(i) sequencing the gene encoding the FcγR2a receptor from the subject's sample; and
(ii) comparing the sequence from the subject's sample to the FcγR2a sequence in a control sample.

3. A composition comprising an anti-FcRn therapy comprising an antibody that blocks the neonatal Fc receptor (FcRn) for use in treating, inhibiting and/or reducing the severity of an immune-mediated disease **characterised by** elevated IgG levels in a subject, wherein the subject has been identified as having the His131 allele of FcγR2a by the assay of any one of claims 1 to 2.

4. The assay of any one of claims 1 to 2, or the composition for use as in claim 3, wherein the subject is human.

5. The assay of claim 1, or the composition for use as in claim 3, wherein the immune-mediated disease is any one or more of monoclonal gammopathy of unknown significance (MGUS), inflammatory bowel disease, pemphigus or pemphigoid, myasthenia gravis, autoimmune hemolytic anemia, Kawasaki's disease, multiple myeloma, rheumatoid arthritis, systemic lupus erythematosus (SLE), cirrhosis of the liver, chronic hepatitis, chronic infection, multiple sclerosis (MS), macroglobulinemia, viral hepatitis, mononucleosis, kidney damage (nephrotic syndrome), celiac disease, allergic reactions, asthma, atopic dermatitis, cancer, idiopathic thrombocytopenic purpura (ITP), autoimmune thrombocytopenia, immune neutropenia, antiphospholipid syndrome, ANCA-associated disease, polymyositis, myasthenia gravis, diabetes, autoimmune thyroiditis, optic neuritis, Graves' opthalmopathy, Guillian-Barre Syndrome, chronic polyneuropathy, acute myeloid leukemia or a combination thereof.

6. The assay of, or the composition for use as in claim 5, wherein the immune-mediated disease is any one or more of inflammatory bowel disease, pemphigus or pemphigoid, myasthenia gravis, autoimmune hemolytic anemia, Kawasaki's disease, rheumatoid arthritis, systemic lupus erythematosus (SLE), chronic hepatitis, multiple sclerosis (MS), idiopathic thrombocytopenic purpura (ITP), antiphospholipid syndrome, ANCA-associated disease, polymyositis, myasthenia gravis, optic neuritis, Guillian-Barre Syndrome, chronic polyneuropathy, or a combination thereof.

7. The assay of, or the composition for use as in claim 5, wherein the immune-mediated disease is inflammatory bowel disease (IBD).

8. The assay of, or the composition for use as in claim 7, wherein the subject
(i) has undergone or is undergoing treatment for inflammatory bowel disease, or
(ii) has not undergone treatment for inflammatory bowel disease.

9. The assay of, or the composition for use as in claim 7, wherein IBD is ulcerative colitis, Crohn's disease, collagenous colitis, lymphocytic colitis, microsopic colitis, ischaemic colitis, diversion colitis, Behcet's disease, indeterminate colitis or a combination thereof.

10. The assay of any one of claims 1 to 2, or the composition for use as in claim 3, wherein the subject is
(a) heterozygous for the His131 allele of FcγR2a, or
(b) homozygous for the His131 allele of FcγR2a.

11. The assay of any one of claims 1 to 2, or the composition for use as in claim 3, wherein the antibody is selected from the group consisting of a monoclonal antibody or a fragment thereof, a polyclonal antibody or a fragment thereof, chimeric antibody, humanized antibody and single chain antibody.

12. The assay of any one of claims 1 to 2, or the composition for use as in claim 3, wherein the sample is tissue or fluid, preferably, wherein the sample is serum or blood, body secretions from the nose, oropharynx, gastrointestinal tract, bile or genitourinary tract, tissue biopsies of any organ, a tissue fluid such as cerebrospinal, occular or joint or a combination thereof.

## Patentansprüche

1. Test, umfassend:
(i) Untersuchen einer biologischen Probe, die von einem Subjekt abgenommen worden ist, von dem vermutet wird, dass es an einer immunvermittelten Krankheit leidet, **gekennzeichnet durch** erhöhte IgG-Spiegel, um das Allel an Position 131 des FcyR2a-Proteins zu bestimmen; und
(ii) Auswählen des Subjekts für eine Therapie, wenn das Subjekt das His131-Allel von FcyR2a exprimiert, wobei die Therapie einen Antikörper umfasst, der den neonatalen Fc-Rezeptor (FcRn) blockiert.

2. Test nach Anspruch 1, wobei das Untersuchen der Probe zum Bestimmen des Allels von FcyR2a Folgendes umfasst:
(i) Sequenzieren des Gens, das für den FcyR2a-Rezeptor codiert, aus der Probe des Subjekts; und
(ii) Vergleichen der Sequenz aus der Probe des Subjekts mit der FcyR2a-Sequenz in einer Kontrollprobe.

3. Zusammensetzung, umfassend eine Anti-FcRn-Therapie umfassend einen Antikörper, der den neonatalen Fc-Rezeptor (FcRn) blockiert, zur Verwendung zum Behandeln, Inhibieren und/oder Reduzieren der Schwere einer immunvermittelten Krankheit, **gekennzeichnet durch** erhöhte IgG-Spiegel in einem Subjekt, wobei das Subjekt durch den Test nach einem der Ansprüche 1 bis 2 als das His131-Allel von FcyR2a aufweisend identifiziert worden ist.

4. Test nach einem der Ansprüche 1 bis 2 oder Zusammensetzung zur Verwendung wie in Anspruch 3, wobei das Subjekt menschlich ist.

5. Test nach Anspruch 1 oder Zusammensetzung zur Verwendung wie in Anspruch 3, wobei die immunvermittelte Krankheit eine oder mehrere ist von monoklonaler Gammopathie unbekannter Signifikanz (MGUS), entzündlicher Darmerkrankung, Pemphigus oder Pemphigoid, Myastenia gravis, autoimunhämolytischer Anämie, Kawasaki-Krankheit, multiplem Myelom, rheumatoider Arthritis, systemischem Lupus erythematodes (SLE), Leberzirrhose, chronischer Hepatitis, chronischer Infektion, multipler Sklerose (MS), Makroglobulinämie, Virushepatitis, Mononukleose, Nierenverletzung (nephrotischem Syndrom), Zöliakie, allergischen Reaktionen, Asthma, atopischer Dermatitis, Krebs, idiopathischer thrombozytopenischer Purpura (ITP), Autoimunthrombozytopenie, Immunneutropenie, Antiphospholipidsyndrom, mit ANCA verbundener Krankheit, Polymyositis, Myasthenia gravis, Diabetes, Autoimmunthyroiditis, optischer Neutritis, endokriner Ophthalmopathie, Guillian-Barre-Syndrom, chronischer Polyneuropathie, akuter myeloischer Leukämie oder einer Kombination davon.

6. Test nach oder Zusammensetzung zur Verwendung wie in Anspruch 5, wobei die immunvermittelte Krankheit eine oder mehrere ist von entzündlicher Darmerkrankung, Pemphigus oder Pemphigoid, Myastenia gravis, autoimunhämolytischer Anämie, Kawasaki-Krankheit, rheumatoider Arthritis, systemischem Lupus erythematodes (SLE), chronischer Hepatitis, multipler Sklerose (MS), idiopathischer thrombozytopenischer Purpura (ITP), Antiphospholipidsyndrom, mit ANCA verbundener Krankheit, Polymyositis, Myasthenia gravis, optischer Neutritis, Guillian-Barre-Syndrom, chronischer Polyneuropathie oder einer Kombination davon.

7. Test nach oder Zusammensetzung zur Verwendung wie in Anspruch 5, wobei die immunvermittelte Krankheit entzündliche Darmerkrankung (IBD) ist.

8. Test nach oder Zusammensetzung zur Verwendung wie in Anspruch 7, wobei das Subjekt
(i) eine Behandlung gegen entzündliche Darmerkrankung durchgemacht hat oder durchmacht oder
(ii) keine Behandlung gegen entzündliche Darmerkrankung durchgemacht hat.

9. Test nach oder Zusammensetzung zur Verwendung wie in Anspruch 7, wobei die IBD Colitis ulcerosa, Morbus Crohn, kollagene Colitis, lymphozytische Colitis, mikroskopische Colitis, ischämische Colitis, Diversionscolitis, Behcet-Krankheit, unbestimmte Colitis oder eine Kombination davon ist.

10. Test nach einem der Ansprüche 1 bis 2 oder Zusammensetzung zur Verwendung wie in Anspruch 3, wobei das Subjekt Folgendes ist
(a) heterozygot für das His131-Allel von FcyR2a oder
(b) homozygot für das His131-Allel von FcyR2a.

11. Test nach einem der Ansprüche 1 bis 2 oder Zusammensetzung zur Verwendung nach Anspruch 3, wobei der Antikörper aus der Gruppe bestehend aus einem monoklonalen Antikörper oder einem Fragment davon, einem polyklonalen Antikörper oder einem Fragment davon, einem chimären Antikörper, einem humanisierten Antikörper und einem Einkettenantikörper ausgewählt ist.

12. Test nach einem der Ansprüche 1 bis 2 oder Zusammensetzung zur Verwendung wie in Anspruch 3, wobei die Probe Gewebe oder Fluid ist, wobei die Probe bevorzugt Serum oder Blut, Körperausscheidungen aus der Nase, dem Mundrachen, dem Magen-Darmkanal, Gallen- oder Urogenitaltrakt, Gewebebiopsien eines Organs, ein Gewebefluid wie ein zerebrospinales, Augen- oder Gelenkfluid oder eine Kombination davon ist.

## Revendications

1. Dosage comprenant :
(i) le dosage d'un échantillon biologique obtenu à partir d'un sujet suspecté d'avoir une maladie à médiation immunitaire **caractérisée par** des taux d'IgG élevés pour déterminer l'allèle à la position 131 de la protéine FcγR2a ; et
(ii) la sélection du sujet pour une thérapie si le sujet exprime l'allèle His131 de FcγR2a, dans lequel la thérapie comprend un anticorps qui bloque le récepteur Fc néonatal (FcRn).

2. Dosage selon la revendication 1, dans lequel le dosage de l'échantillon pour déterminer l'allèle de FcγR2a comprend :
(i) le séquençage du gène codant pour le récepteur FcγR2a à partir de l'échantillon du sujet ; et
(ii) la comparaison de la séquence de l'échantillon du sujet à la séquence de FcγR2a dans un échantillon témoin.

3. Composition comprenant une thérapie anti-FcRn comprenant un anticorps qui bloque le récepteur Fc néonatal (FcRn) pour utilisation dans le traitement, l'inhibition et/ou la réduction de la sévérité d'une maladie à médiation immunitaire **caractérisée par** des taux d'IgG élevés chez un sujet, où le sujet a été identifié comme ayant l'allèle His131 de FcγR2a par le dosage selon l'une quelconque des revendications 1 à 2.

4. Dosage selon l'une quelconque des revendications 1 à 2, ou composition pour utilisation selon la revendication 3, où le sujet est humain.

5. Dosage selon la revendication 1, ou composition pour utilisation selon la revendication 3, où la maladie à médiation immunitaire est l'un ou plusieurs quelconques parmi une gammapathie monoclonale de signification inconnue (GMSI), une maladie inflammatoire chronique de l'intestin, le pemphigus ou le pemphigoïde, la myasthénie grave, l'anémie hémolytique auto-immune, la maladie de Kawasaki, le myélome multiple, la polyarthrite rhumatoïde, le lupus érythémateux disséminé (LED), la cirrhose du foie, l'hépatite chronique, une infection chronique, la sclérose en plaques (SP), la macroglobulinémie, une hépatite virale, une mononucléose, des lésions rénales (syndrome néphrotique), la maladie cœliaque, des réactions allergiques, l'asthme, la dermatite atopique, le cancer, le purpura thrombopénique idiopathique (PTI), la thrombocytopénie auto-immune, la neutropénie immunitaire, le syndrome des antiphospholipides, une maladie associée à ANCA, la polymyosite, la myasthénie grave, le diabète, la thyroïdite auto-immune, la névrite optique, l'ophtalmopathie de Graves, le syndrome de Guillain-Barré, une polyneuropathie chronique, une leucémie myéloïde aiguë ou une combinaison de ceux-ci.

6. Dosage de, ou composition pour utilisation selon, la revendication 5, où la maladie à médiation immunitaire est l'un ou plusieurs quelconques parmi une maladie inflammatoire chronique de l'intestin, le pemphigus ou le pemphigoïde, la myasthénie grave, l'anémie hémolytique auto-immune, la maladie de Kawasaki, la polyarthrite rhumatoïde, le lupus érythémateux disséminé (LED), une hépatite chronique, la sclérose en plaques (SP), le purpura thrombopénique idiopathique (PTI), le syndrome des antiphospholipides, une maladie associée à ANCA, la polymyosite, la myasthénie grave, la névrite optique, le syndrome de Guillain-Barré, une polyneuropathie chronique, ou une combinaison de ceux-ci.

7. Dosage de, ou composition pour utilisation selon, la revendication 5, où la maladie à médiation immunitaire est une maladie inflammatoire chronique de l'intestin (MICI).

8. Dosage de, ou composition pour utilisation selon, la revendication 7, où le sujet
(i) a reçu ou reçoit un traitement pour une maladie inflammatoire chronique de l'intestin, ou
(ii) n'a pas reçu un traitement pour une maladie inflammatoire chronique de l'intestin.

9. Dosage de, ou composition pour utilisation selon, la revendication 7, où la MICI est la recto-colite hémorragique, la maladie de Crohn, la colite collagénique, la colite lymphocytaire, la colite microscopique, la colite ischémique, la colite de diversion, la maladie de Behçet, une colite indéterminée ou une combinaison de celles-ci.

10. Dosage selon l'une quelconque des revendications 1 à 2, ou composition pour utilisation selon la revendication 3, où le sujet est
(a) hétérozygote pour l'allèle His131 de FcγR2a, ou
(b) homozygote pour l'allèle His131 de FcγR2a.

11. Dosage selon l'une quelconque des revendications 1 à 2, ou composition pour utilisation selon la revendication 3, où l'anticorps est choisi dans le groupe constitué d'un anticorps monoclonal ou un fragment de celui-ci, un anticorps polyclonal ou un fragment de celui-ci, un anticorps chimérique, un anticorps humanisé et un anticorps monocaténaire.

12. Dosage selon l'une quelconque des revendications 1 à 2, ou composition pour utilisation selon la revendication 3, où l'échantillon est un tissu ou un fluide, de préférence, où l'échantillon est du sérum ou du sang, des sécrétions corporelles provenant du nez, de l'oropharynx, ddu tube digestif, des voies biliaires et de l'appareil génito-urinaire, des biopsies de tissu d'un organe quelconque, un fluide tissulaire tel que le liquide céphalo-rachidien, un fluide oculaire ou articulaire ou une combinaison de ceux-ci.
